# EUROPEAN PATENT SPECIFICATION

(11) **EP 2 640 419 B1**
(45) Date of publication and mention of the grant of the patent: **05.12.2018**
(21) Application number: 11781825.2
(22) Date of filing: 15.11.2011
(51) Int. Cl.: A61K 39/155, A61P 31/12

(54) **RESPIRATORY SYNCYTIAL VIRUS VACCINE**
IMPFSTOFF GEGEN RESPIRATORISCHES SYNZYTIALVIRUS
VACCIN CONTRE LE VIRUS RESPIRATOIRE SYNCYTIAL

(30) Priority: 15.11.2010 GB 201019240; 15.11.2010 US 458012 P
(43) Date of publication of application: 25.09.2013
(73) Proprietor: VIB VZW, 9052 Gent (BE); Universiteit Gent, 9000 Gent (BE)
(72) Inventor: SAELENS, Xavier, 8900 Ieper (BE); SCHEPENS, Bert, 9031 Drongen (BE); FIERS, Walter, 9070 Destelbergen (BE)
(74) Representative: Comoglio, Elena
(86) International application number: PCT/EP2011/070161
(87) International publication number: WO 2012/065997

(56) References cited:
- US-A1- 2007 184 069
- PRINGLE C R ET AL: "IMMUNOGENICITY AND PATHOGENICITY OF A TRIPLE TEMPERATURE-SENSITIVE MODIFIED RESPIRATORY SYNCYTIAL VIRUS IN ADULT VOLUNTEERS", VACCINE, ELSEVIER LTD, GB, vol. 11, no. 4, 1 March 1993 (1993-03-01), pages 473-478, XP001018229, ISSN: 0264-410X, DOI: 10.1016/0264-410X(93)90290-E
- POWER U F ET AL: "Induction of Protective Immunity in Rodents by Vaccination with a Prokaryotically Expressed Recombinant Fusion Protein Containing a Respiratory Syncytial Virus G Protein Fragment", VIROLOGY, ACADEMIC PRESS,ORLANDO, US, vol. 230, no. 2, 14 April 1997 (1997-04-14), pages 155-166, XP004452312, ISSN: 0042-6822, DOI: 10.1006/VIRO.1997.8465
- BASTIEN N ET AL: "Complete protection of mice from respiratory syncytial virus infection following mucosal delivery of synthetic peptide vaccines", VACCINE, ELSEVIER LTD, GB, vol. 17, no. 7-8, 26 February 1999 (1999-02-26), pages 832-836, XP004154821, ISSN: 0264-410X, DOI: 10.1016/S0264-410X(98)00267-9
- SINGH ET AL: "Immunogenicity and efficacy of recombinant RSV-F vaccine in a mouse model", VACCINE, ELSEVIER LTD, GB, vol. 25, no. 33, 24 July 2007 (2007-07-24) , pages 6211-6223, XP022168621, ISSN: 0264-410X, DOI: 10.1016/J.VACCINE.2007.05.068
- Edward E. Walsh: "Respiratory Syncytial Virus Vaccine", Encyclopedia of Molecular Cell Biology and Molecular Medicine, 2nd Edition, vol. 12 2005, pages 297-322, XP002670236, ISBN: 3-527-30649-8 Retrieved from the Internet: URL:http://onlinelibrary.wiley.com/doi/10. 1002/3527600906.mcb.200500028/pdf [retrieved on 2012-02-23]
- WOO W-P ET AL: "Hepatitis B Surface Antigen Vector Delivers Protective Cytotoxic T-Lymphocyte Responses to Disease-Relevant Foreign Epitopes", JOURNAL OF VIROLOGY, THE AMERICAN SOCIETY FOR MICROBIOLOGY, US, vol. 80, no. 8, 1 April 2006 (2006-04-01), pages 3975-3984, XP003001186, ISSN: 0022-538X
- SCHMIDT A C ET AL: "Mucosal immunization of Rhesus monkeys against respiratory syncytial virus subgroups A and B and human parainfluenza virus type 3 by using a live cDNA-derived vaccine based on a host range-attenuated bovine parainfluenza virus type 3 vector backbone", JOURNAL OF VIROLOGY, THE AMERICAN SOCIETY FOR MICROBIOLOGY, US, vol. 76, no. 3, 1 February 2002 (2002-02-01), pages 1089-1099, XP002978183, ISSN: 0022-538X, DOI: 10.1128/JVI.76.23.12355-12359.2002
- YOSHIHIKO MURATA: "Respiratory Syncytial Virus Vaccine Development", CLINICS IN LABORATORY MEDICINE, vol. 29, no. 4, 1 December 2009 (2009-12-01), pages 725-739, XP55008639, ISSN: 0272-2712, DOI: 10.1016/j.cll.2009.07.004

## Description

The present invention relates to a vaccine for use in a method of vaccination against Respiratory Syncytial Virus (RSV), as defined in appended claim 1. More specifically, the invention relates to a recombinant subunit vaccine as defined in appended claim 1, for use in a method of vaccination. The ectodomain of SH is referred to as SHe. Preferably said ectodomain is presented as an oligomer, even more preferably as a pentamer. The invention relates further to antibodies, specific for said ectodomain, for use in protecting a subject against RSV infection and/or in the treatment of an infected subject.

The scope of the invention is defined by the appended claims.

RSV infection is the leading cause of infant hospitalization in industrialized countries.

Following primary RSV infection, which generally occurs under the age of 2 years, immunity to RSV remains incomplete, and reinfection can occur. Furthermore, RSV can cause serious disease in the elderly and is in general associated with higher mortality than influenza A in non- pandemic years (Falsey et al., 1995). The WHO-estimated global annual infection rate in the human population is estimated at 64 million cases, with a mortality figure of 160000; in the US alone, from 85000 to 144000 infants are hospitalized each year as a consequence of RSV infection (http://www.who.int/vaccine_research/diseases/ari/en/index2.html update 2009).

RSV belongs to the family *Paramyxoviridae,* subfamily *Pneumovirinae,* genus *Pneumovirus*; in human, there are two subgroups, A and B. Apart from the human RSV, there is a bovine variant. The genome of human RSV is approximately 15200 nucleotides long and is a negative-sense RNA molecule. The RSV genome encodes 11 known proteins: Glycoprotein (G), Fusion protein (F), Small hydrophobic protein (SH), Nucleoprotein (N), Phosphoprotein (P), Large protein (L), Matrix protein (M), M2 ORF-1 protein (M2-1), M2 ORF-2 protein (M2-2), Nonstructural protein 1 (NS1) and Nonstructural protein 2 (NS2). G, F and SH are transmembrane surface proteins; N, P, L, M, M2-1 are nucleocapsid associated proteins and NS1 and NS2 are non-structural proteins. The status of M2-2 as a structural or nonstructural protein is unknown. (Hacking and Hull, 2002). The RSV subgroups show differences in the antigenic properties of the G, F, N and P proteins (Ogra, 2004). RSV infection is followed by the formation of specific IgG and IgA antibodies detectable in the serum and some other body fluids. Several studies have demonstrated that antibody responses are mainly directed to the major RSV transmembrane proteins F and G; only F- and G-specific antibodies are known to have in vitro RSV-neutralizing activity. Antibody responses to the F protein are often cross-reactive between the A and B subgroups, whereas antibody responses to the G protein are subgroup specific (Orga, 2004). Contrary to F and G, the transmembrane protein SH is considered as non-immunogenic (Gimenez et al., 1987; Tsutsumi et al., 1989) and in some vaccine candidates, SH has even been deleted in order to obtain a non-revertible attenuated vaccine (Karron et al., 2005).

Pringle C.R. et al., Vaccine, Elsevier Ltd, GB, vol. 11, n° 4, pages 473-478, discloses an immunogenic composition comprising a RSV with three temperature sensitive mutations.

Singh et al., Vaccine, Elsevier Ltd, GB, vol. 25, n° 33, pages 6211-6223, discloses an immunogenic composition comprising residues 412-524 of RSV-F.

Edward E. Walsh: "Respiratory Syncytial Virus Vaccine", Encyclopedia of Molecular Cell Biology and Molecular Medicine, 2nd edition, vol. 12, 2005, pages 297-322, states that T- and B-cell epitopes have been identified in the SH protein of RSV, but contains no reference to their location within the SH sequence.

Development of vaccines to prevent RSV infection has been complicated by the fact that host immune responses appear to play a significant role in the pathogenesis of the disease. Early attempts at vaccinating children with formalin-inactivated RSV showed that vaccinated children experienced a more severe disease on subsequent exposure to the virus as compared to the unvaccinated controls (Kapikian et al., 1969). Live attenuated vaccines have been tested, but show often over- or underattenuation in clinical studies (Murata, 2009).

Subunit vaccines, using one immunogenic protein or a combination of immunogenic proteins are considered safer, because they are unable to revert or mutate to a virulent virus. Candidate vaccines based on purified F protein have been developed and were tested in rodents, cotton rats, and humans, and were shown to be safe, but only moderately immunogenic (Falsey and Walsh, 1996; Falsey and Walsh, 1997; Groothuis et al. 1998). In a similar vein, clinical trials with a mixture of F-, G- and M-proteins have been discontinued in phase II (ADISinsight Clinical database). An alternative approach consisted of a recombinant genetic fusion of the antigenic domain of human RSV G protein to the C-terminal end of the albumin-binding domain of the streptococcal G protein (BBG2Na; Power et al., 2001). BBG2Na was investigated up to a phase III clinical trial in healthy volunteers, but the trial had to be stopped due to the appearance of unexpected type 3 hypersensitivity side effects (purpura) in some immunized volunteers (Meyer et al., 2008).

A recent development is the use of chimeric recombinant viruses as vector for RSV antigens. A chimeric recombinant bovine/human parainfluenzavirus type 3 (rB/HPIV-3) was engineered by substituting in a BPIV-3 genome the F and HN genes by the homologous genes from HPIBV-3. The resulting chimeric rB/HPIV-3 strain was then used to express the HRSV F and G genes (Schmidt et al., 2002). This vaccine is currently under clinical investigation.

There are only a limited number of prevention and treatment options available for severe disease caused by RSV. The most widely used intervention is based on passive immunoprophylaxis with a humanized monoclonal antibody that is derived from mouse monoclonal antibody 1129 (Beeler and van Wyke Coelingh, 1989). This antibody is specific for RSV F protein and neutralizes subgroup A and B viruses. The recombinant humanized antibody 1129 is known as palivizumab (also known as Synagis) and is used for prophylactic therapy of infants that are at high risk of developing complications upon RSV infection. The antibody is administered intramuscularly on a monthly basis in order to lower the risk of RSV infection in infants at risk due to prematurity, chronic lung disease, or hemodynamically significant congenital heart disease (Bocchini et al., 2009). Some studies have reported acceptable cost-effectiveness ratios for RSV prophylaxis with palivizumab (Prescott et al., 2010).

As there is no approved vaccine on the market, there is still an unmet need for development and availability of a safe and efficient RSV vaccine. Surprisingly, we found that the extracellular part (ectodomain) of the small hydrophobic protein SH, referred to as SHe, can be used safely for vaccination against RSV infection, especially when it is presented on a carrier as an oligomer preferably as a pentamer. Furthermore, polyclonal or monoclonal antibodies, directed against SHe, can also be used prophylactically or therapeutically for prevention or treatment of RSV infection, respectively.

A first aspect of the invention is a vaccine as defined in appended claim 1 for use in a method of vaccination against RSV. In one preferred embodiment, RSV is either a human subgroup A or a human subgroup B strain, in another preferred embodiment RSV is bovine RSV. The SH protein is known to the person skilled in the art, and contains 64 (RSV subgroup A), 65 (RSV subgroup B) amino acid residues or 81, 77 or 72 amino acid residues for bovine RSV. In one preferred embodiment, the ectodomain of SH (SHe) consists of the 23 carboxy terminal amino acids for subgroup A (SEQ ID N° 1), and of the 24 carboxy terminal amino acids for subgroup B (SEQ ID N° 2). Preferably the sequence of the ectodomain is selected from the group consisting of SEQ ID N° 1 (ectodomain subgroup A) and SEQ ID N° 2 (ectodomain subgroup B). A carrier molecule is a molecule that is heterologous to the SH protein; a carrier can be any carrier known to the person skilled in the art as suitable for the presentation of an antigen and includes, but is not limited to virus like particles such as HBcore (Whitacre et al., 2009), and other VLPs derived from assembling virus capsid or coat proteins. Any other molecular construct can also be used, provided it can present efficiently antigens to the immune system, such as the pentameric Cartilage Oligomeric Matrix Protein (comp; McFarlane et al., 2009), Thromobospondins 3 and 4 (Malashkevich et al., 1996), the B subunit of bacterial AB5 type toxins (e.g. subunit of Cholera toxin or E. coli heat labile toxin; Williams et al., 2006), a pentameric tryptophan-zipper (Liu et al., 2004), a pentameric phenylalanine-zipper (Liu et al., 2006) or a tetrameric GCN4-derived leuzine zipper (tGCN4, De Filette et al., 2008) and Lpp-56 (Shu et al, 2000). The carrier can be of proteinaceous nature, as well as of non-proteinaceous nature. Examples of non-proteinaceous nature are, as a non-limiting example, liposomes, CLIPS™ constructs (Timmerman et al, 2007) and trimethyl chitosan (Slütter et al., 2010).

Preferably, said carrier presents the SHe as an oligomer, even more preferably as a pentamer, by presenting multiple SHe molecules on one scaffold, by presenting one SHe on a multimerizing scaffold, or by a combination of both. The SHe oligomer may be presented as a linear repeated structure, or as individual SHe units forming an oligomeric complex, or as a combination of both. Preferably, said carrier is an oligomeric carrier (dimeric, up to decameric). Even more preferably, said carrier is a pentameric carrier. In one specific embodiment, the transmembrane domain of SH, preferably without the cytoplasmic domain, can be used as oligomerizing domain, further fused or linked to a carrier. Not all carrier molecules should be loaded by SHe; indeed, as a non-limiting example, one can imagine that only 5 units of a hexameric carrier are loaded with SHe, thereby presenting a pentameric SHe complex on a hexameric carrier complex. The ectodomain can be genetically linked to the carrier, forming a fusion protein; both domains may be directly fused, or they may be linked by a hinge sequence or a spacer sequence. As used here, in a genetically fused construct, a hinge sequence is an amino acid sequence that links two domains together; preferably said sequence links the two domains in a flexible way; preferably said hinge sequence is shorter than 150 amino acids, even more preferably shorter than 100 amino acids, even more preferably shorter than 50 amino acids, most preferably shorter than 20 amino acids. A spacer as used here indicates a short hinge sequence shorter than 15 amino acids. In a preferred embodiment, a hinge sequence comprises the sequence (Gly-Ser)ₙ with n equal to one, 2, 3, ...20. In another preferred embodiment, the hinge of immunoglobulin genes, such as the hinge region of human lgG1 is used as hinge sequence. In case of a genetic linkage, said linkage may occur at the amino terminal end of the SHe, as well as at the carboxy terminal end.

Alternatively, the ectodomain is chemically linked to the carrier. Chemical linkage is known to the person skilled in the art, and includes but is not limited to peptides that are conjugated to the carrier by covalently joining peptides to reactive sites on the surface of the carrier. The resulting structure is a conjugate. A reactive site on the surface of the carrier is a site that is chemically active or that can be activated and is sterically accessible for covalent joining with a peptide. A preferred reactive site is the epsilon nitrogen of the amino acid lysine. Covalently joined refers to the presence of a covalent linkage that is stable to hydrolysis under physiological conditions. Preferably, the covalent linkage is stable to other reactions that may occur under physiological conditions including adduct formation, oxidation, and reduction. Often the linkage of an antigenic peptide to a carrier is achieved using bifunctional reagents (Hermanson, 1996). Any suitable residue in the SHe may be used for linkage to the chemical carrier; preferably, SHe is linked to the carrier by its amino terminal or carboxy terminal end. In still another embodiment, the ectodomain is linked to the carrier by a noncovalent interaction, such as, but not limited to hydrophobic interactions, cooperative H-bond interactions, or Van der Waals interactions.

The vaccine can be administrated to the subject to be treated by any route known to the person skilled in the art, including but not limited to intranasal, intraperitonial, intramuscular and intradermal administration. Preferably, there is no enhancement of the disease symptoms upon RSV infection after vaccination. The vaccine can be for animal or for human use. A preferred animal use is for protection of cattle or other Bovidae by vaccination against bovine respiratory viruses related to human RSV, such as but not limited to Bovine RSV. Protection against RSV infection covers both prophylactic and therapeutic uses. More particularly, a preferred use of the vaccine is for prophylactic purposes. "Preparation of a vaccine" as used here means that the immunogenic composition of the vaccine according to the invention may be optimized by addition of suitable excipients, or it may be formulated for, as a non-limiting example, increasing the shelf life or improving the pharmaceutical characteristics of the vaccine.

As a non-limiting example, immunogenic compositions comprising SHe of RSV subgroup A and SHe of RSV subgroup B may be mixed to obtain a vaccine with a broader specificity. Said vaccine can be for human or for veterinary use. Apart from the immunogenic composition, the vaccine comprises one or more other compounds, such as an adjuvant. Preferably the vaccine is a vaccine for the protection of humans against RSV infection, or, in animals, against animal respiratory viruses related to human RSV, such as but not limited to bovine RSV.

The immunogenic composition of the vaccine of the invention may be used for the detection and/or purification of antibodies, directed against the ectodomain of RSV. Such antibodies may be isolated after vaccinating a subject with the immunogenic composition of the invention; alternatively, similar antibodies and/or antibody producing cells can also be obtained from an RSV-infected human or animal subject, and, after proper development known in the art, used for production of SHe-specific antibodies, preferably human-type antibodies which can be used for prophylactic or therapeutic purposes as described above.

Another aspect of the invention is a RSV inhibiting monoclonal antibody, directed against the ectodomain of the RSV SH-protein. RSV inhibiting, as used here, means that, upon infection, the lung virus titer is lower in treated animals compared to the non-treated animals, as measured in a suitable animal model. Preferably, said monoclonal antibody is a human or humanized monoclonal antibody.

Still another aspect of the invention is a pharmaceutical composition, comprising a monoclonal antibody directed against the ectodomain of the RSV SH-protein, according to the invention. Indeed, an organ of an immunized non-human animal, preferably the spleen of said animal, or a blood sample from an immunized animal or human subject, can be used as starting material for the production of monoclonal antibodies and derivatives such as, but not limited to single chain antibodies, multivalent antibodies, or antibodies linked to antiviral compounds. Said monoclonal antibodies and derivatives are used for passive immunization, or for treatment of RSV infection.

### BRIEF DESCRIPTION OF THE FIGURES

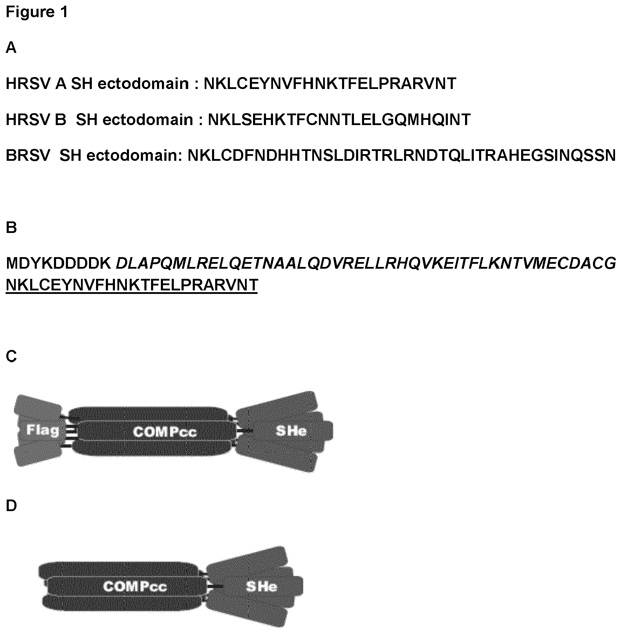

**Figure 1****:** A, The amino acid sequences of the subtype A human RSV (hRSV) SH ectodomain (SEQ ID N° 1), of the subtype B human RSV SH ectodomain (SEQ ID N° 2) and of the bovine RSV (bRSV) SH ectodomain (SEQ ID N° 17). B, The amino acid sequence of Flag-COMPcc- She (SEQ ID N° 35). The first nine amino acids represent the N-terminal Flag-tag. The amino acids (AA) in italic font represent the coiled coil domain of rat COMP (AA 25-72). The underlined AA represent the ectodomain of the RSV A small hydrophobic protein (SHe). C,
   Schematic representation of Flag-COMPcc-SHe pentameric protein. D, Schematic representation of COMPcc-SHe pentameric protein.

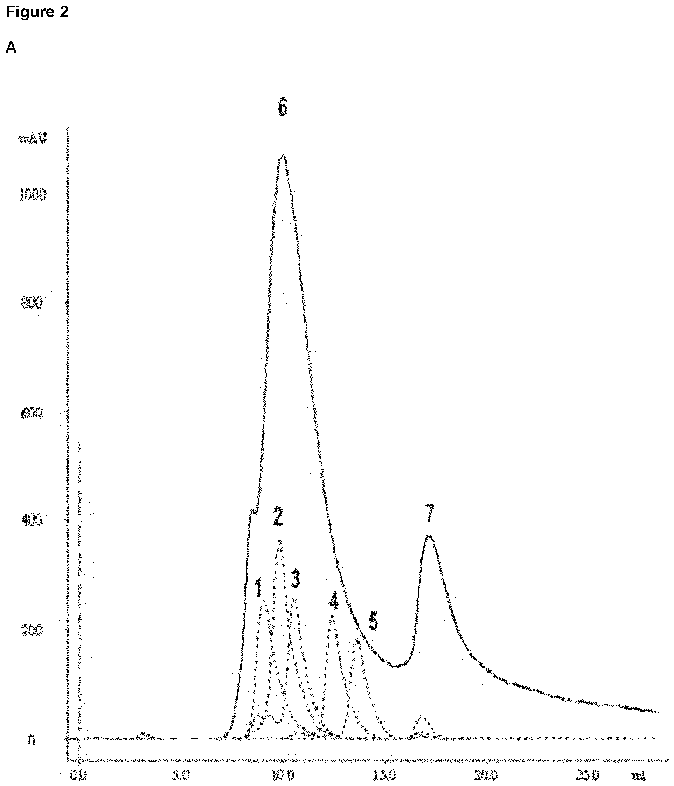

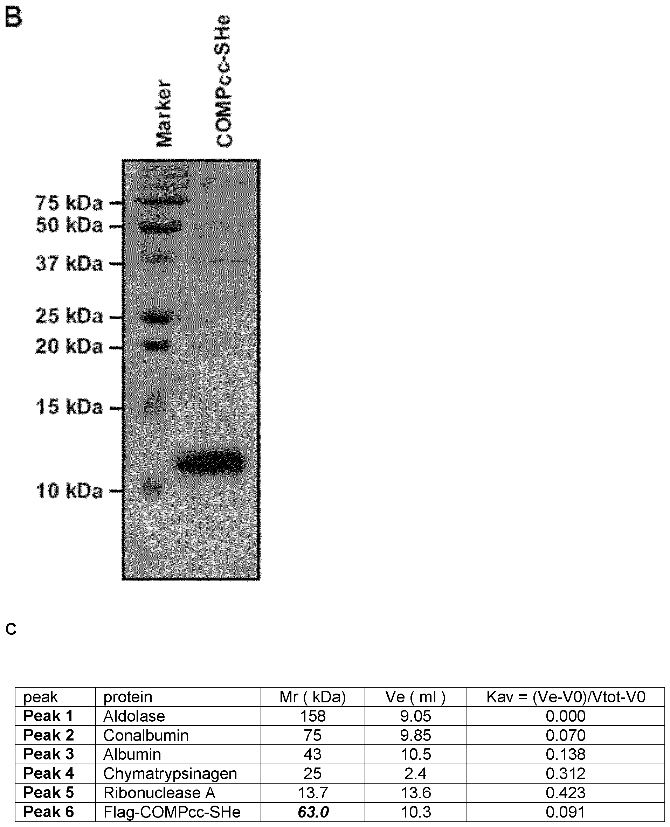

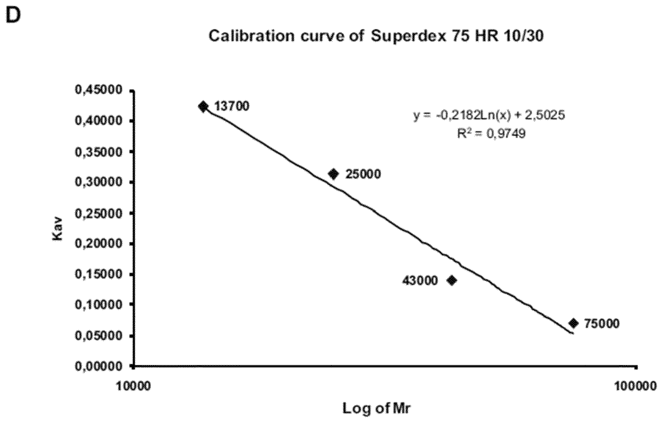

**Figure 2****:** Purification and determination of the relative molecular mass of Flag-COMPcc-SHe. A, Elution curves of aldolase (1), conalbumin (2), albumin (3), chymatrysinagen (4), ribonuslease A (5) and Flag-COMPcc-SHe (6 en 7) upon gelfiltration on a superdex 75 column. B, Coomassie blue staining of a SDS-PAGE analysis of Flag-COMPcc-SHe after gelfiltration (peak 6 of panel A). C, overview of the proteins used to calibrate the gelfiltration column, their relative molecular weight (Mr), the Volume at which they eluted from the column (Ve) and the calculated Kav (Kav = (Ve-V0)/(Vtot-V0), with V0 = the column void volume = 9.05 and Vtot = the column bed volume = 19.816). The Mr of Flag-COMPcc-SHe present in peak 6 was calculated based on its Ve and the calibration curve presented in panel D. D, The calibration curve of the superdex 75 gelfiltration column used to purify pentameric Flag- COMPcc-SHe.

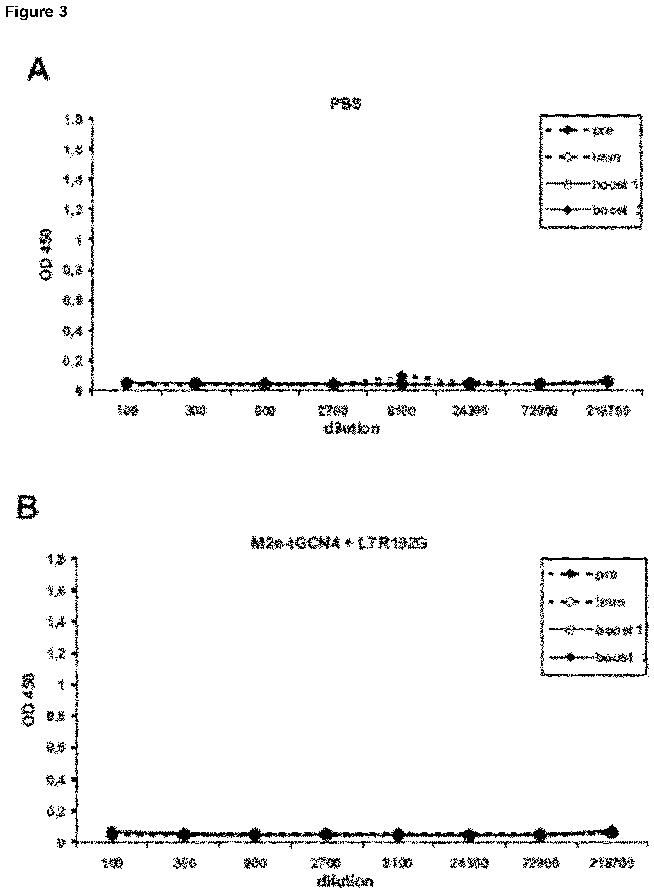

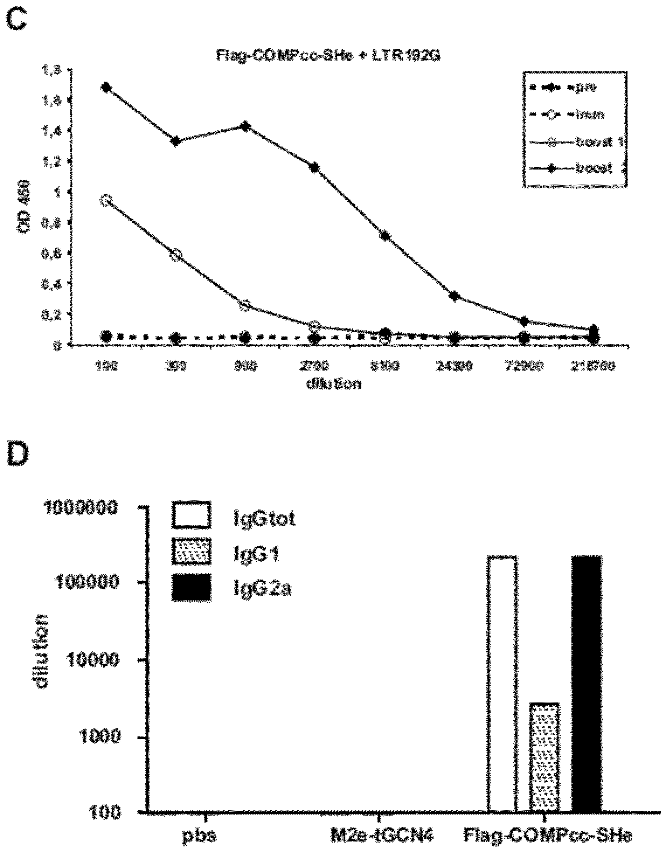

**Figure 3****:** Vaccination of Balb/c mice with Flag-COMPcc-SHe in combination with LTR192G induces She-specific antibodies. A, B and C, ELISA based determination of the SHe peptide specific IgG antibodies titers present in the pooled sera of mice after the first, second or third immunization with the indicated vaccines. D, SHe peptide specific IgG, lgG1 and lgG2a antibodies present in the pooled sera of mice that were vaccinated with PBS, M2e-1GCN4/LTR192G or Flag-COMPcc-SHe/LTR192G.

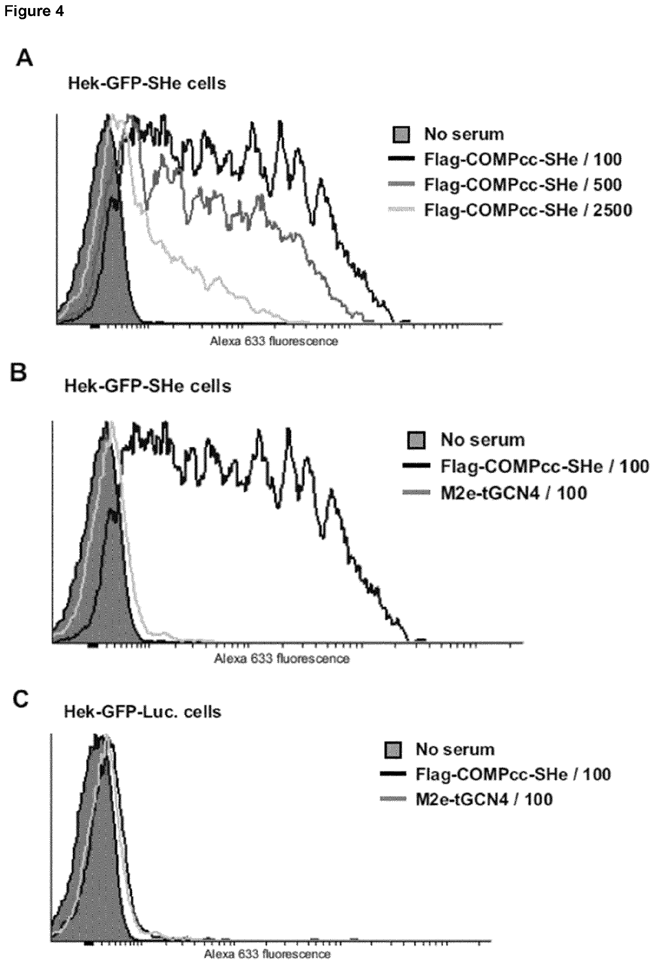

**Figure 4****:** Flag-COMPcc-SHe vaccination, as in legend of Figure 3, induces antibodies that can recognize the SH-ectodomain on the surface of cells. A, Flow cytometric analysis of GFP and RSV SH-expressing HEK293T cells stained by different dilutions of serum of Flag-COMPcc-SHe vaccinated mice. B, Flow cytometric analysis of GFP and RSV SH-expressing HEK cells stained by serum from Flag-COMPcc-SHe or M2e-tGCN4 (negative control) vaccinated mice. C, Flow cytometric analysis of GFP and Luciferase expressing HEK cells stained by serum from Flag-COMPcc-SHe or M2e-tGCN4 vaccinated mice.

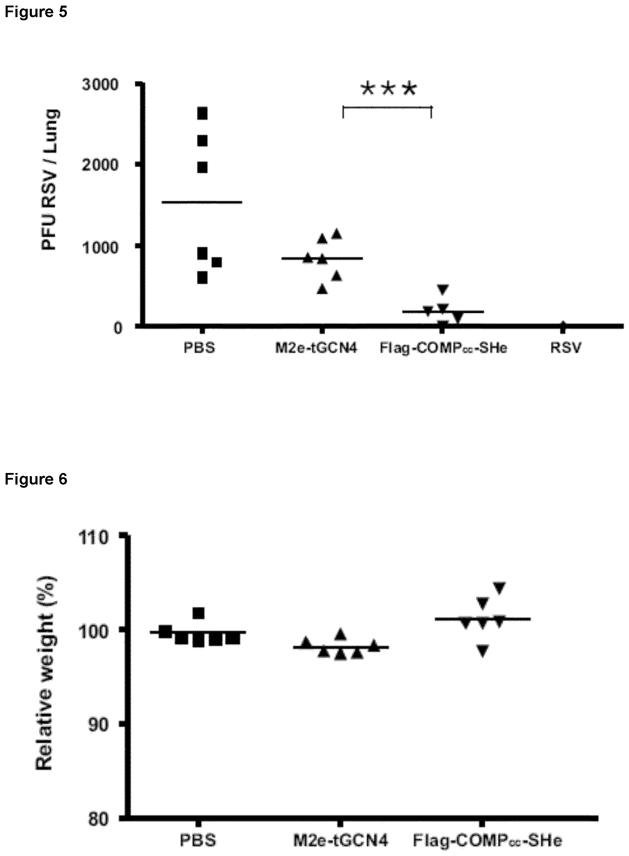

**Figure 5****:** Flag-COMPcc-SHe vaccination inhibits RSV replication. Four days after challenge mice of the indicated groups were sacrificed to determine viral lung titer by plaque assay. The graph shows the number of plaque forming units per lung of each mouse. The detection limit of the plaque assay is 10 PFU per lung. The difference in RSV lung titer between the Flag- COMPcc-SHe vaccinated and the M2e-tGCN4 vaccinated mice was highly significant (*** p≤ 0.0005).
**Figure 6****:** Flag-COMPcc-SHe vaccination does not induce enhanced disease upon RSV infection. The graph shows the relative body weight of each mouse, calculated as the ratio between the weight at the day of sacrifice (four days after infection) and the weight at the day of viral infection, multiplied by 100.

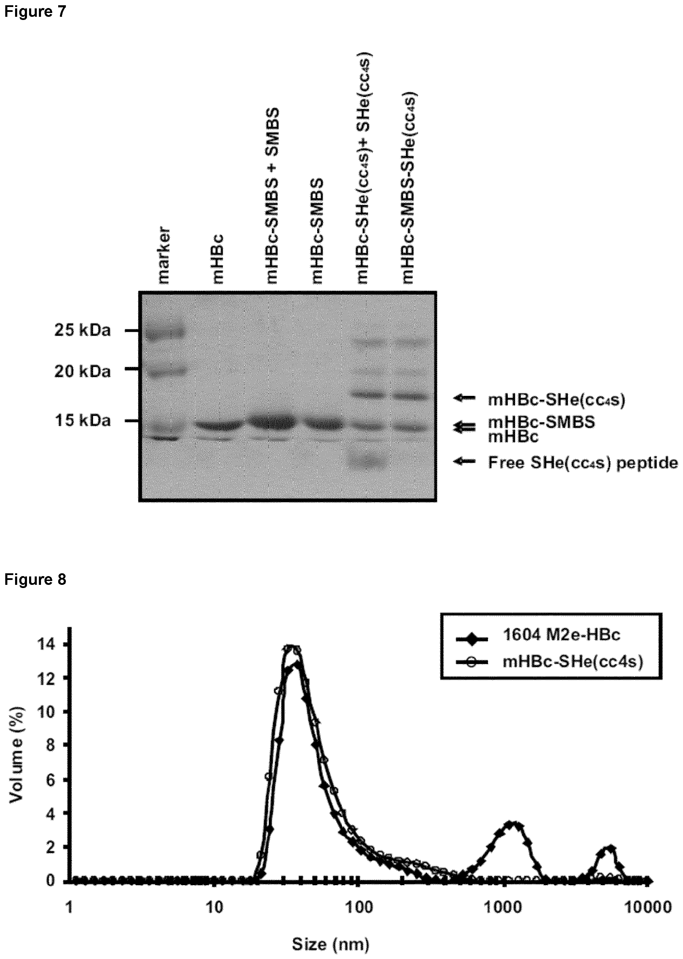

**Figure 7****:** Chemical linkage of SHe(cc4s) peptides to the immunodominant loops of mHBc virus like particles. Coomassie blue stained SDS-PAGE analysis of mHBc at the different stages of chemical linkage as indicated above the gel: mHBc = purified mHBc, mHBc-SMBS + sMBS = mHBc after addition of the chemical linker Sulfo-MBS, mHBc-SMBS = mHBc-SMBS after size exlusion chromatography, mHBC-SHe(cc4s) + SHe(cc4s) = purified mHBc-SMBS after incubation with SHe(cc4s) peptide, mHBC-SHe(cc4s)= SHe linked to mHBc VLP's after purification by size exclusion chromatography.
**Figure 8****:** mHBc-SHe(cc4s) retains its VLP conformation. The graph represents the size distribution of mHBc-SHe(cc4s) and the well described M2e-mBHc VLP 1604 as determined by dynamic light scattering. The size distribution is expressed in function of the Volume.

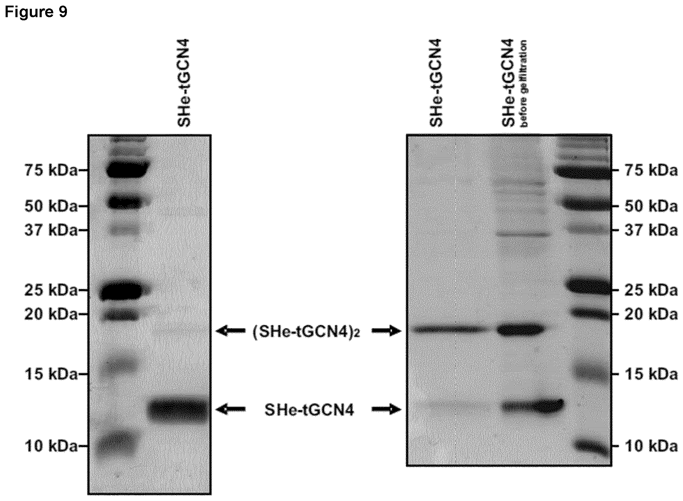

**Figure 9****:** Purification of SHe-tGCN4. SDS-PAGE analysis followed by coomassie blue staining of SHe-tGCN4 after purification by a series of column chromatographic steps: anion exchange, hydrophobic interaction and gelfiltration chromatography. The left and right panels represent SDS-PAGE analysis under reducing (in the presence of beta-mercaptoethanol) or non-reducing (in the absence of beta-mercaptoethanol) respectively. The arrows indicate monomeric and dimeric SHe-tGCN4 proteins.

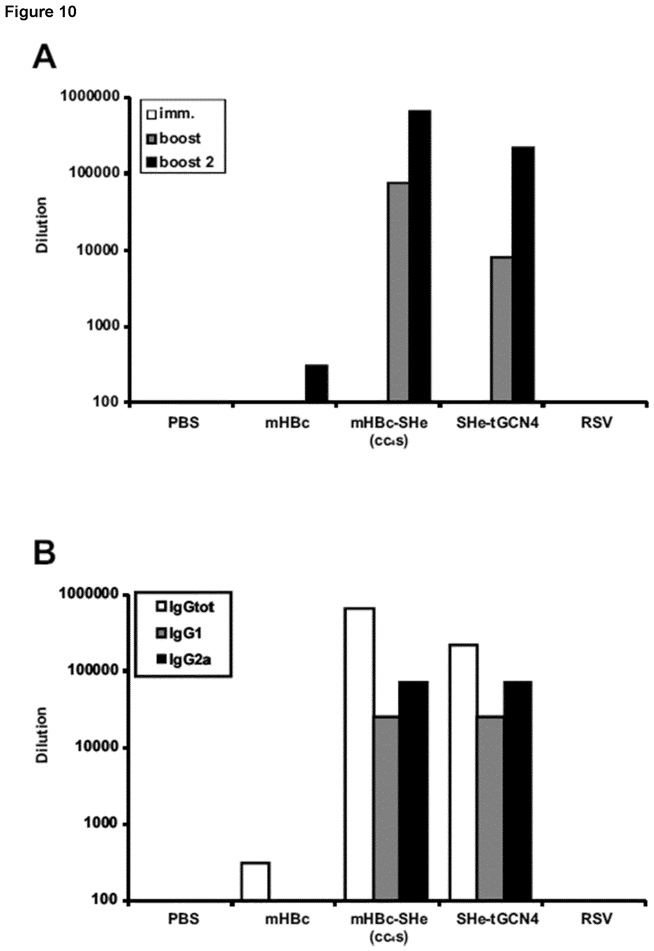

**Figure 10****:** Both SHe-tGCN4 and mHBc-SHe(cc4s) vaccination induce SHe peptide-specific antibodies. A, The figure represents the titers of SHe specific IgG antibodies present in the pooled sera of mice of the indicated groups after the first immunization, the first boost immunization (boost) and the second boost immunization (boost 2), as analyzed by SHe peptide ELISA. B, The figure represents the titers of SHe-specific IgG, lgG1 and lgG2a antibodies present in the pooled sera of mice of the indicated groups after the second boost immunization, as determined by peptide ELISA.

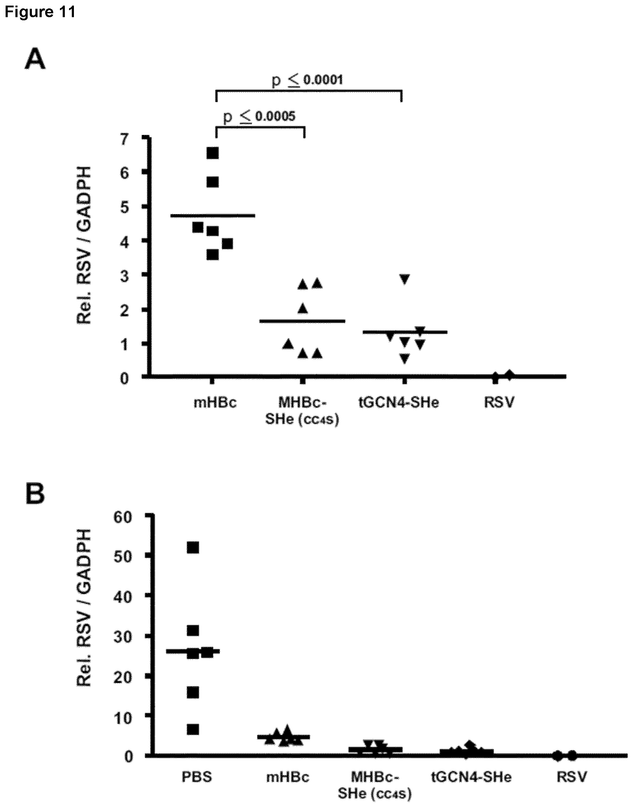

**Figure 11****:** Both SHe-tGCN4 and mHBc-SHe(cc4s) vaccination decrease pulmonary RSV replication. Three day after challenge the mice were sacrificed to determine the viral lung titer by QRt-PCR. The upper graph represents the relative expression of genomic RSV RNA, normalized to the GADPH mRNA levels present in the samples of each mouse in the indicated
   groups. The statistical differences between the vaccinated groups are indicated. The lower panel (B) is identical to the upper panel (A) but also includes the results from the PBS vaccinated mice.

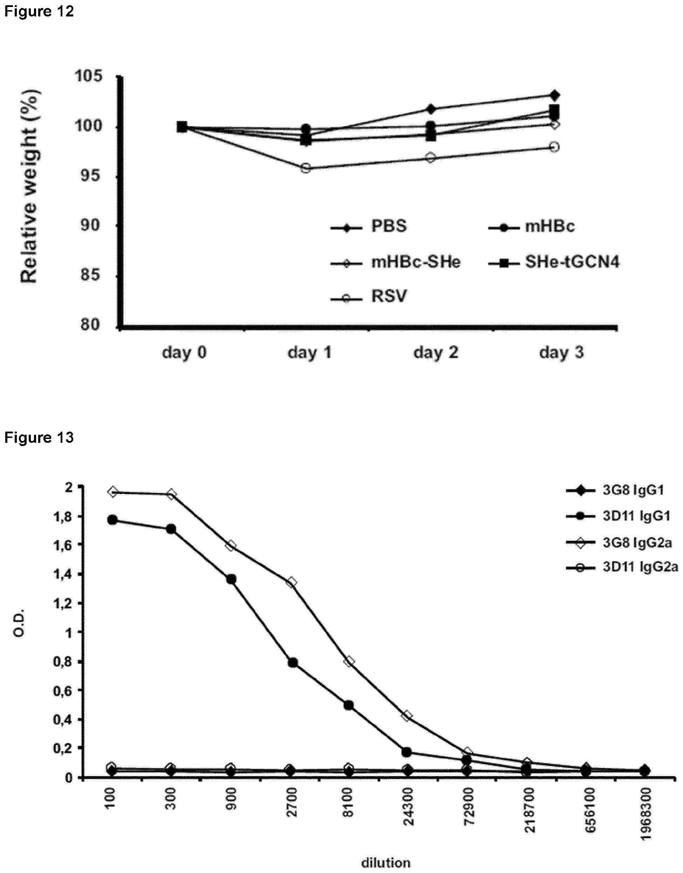

**Figure 12****:** Neither mHBc-SHe(cc4s) nor tGCN4-SHe vaccination induces enhanced disease upon RSV infection. The figure shows the average relative bodyweight of each indicated group of mice, calculated as the ratio between the weight at the indicated day and the weight at the day of infection (day 0), multiplied by 100.
**Figure 13****:** 3D11 and 3G8 are two SHe specific monoclonal Abs of respectively the IgG1 and IgG2a subtype. The graph shows the binding of dilution series of 1µg/µl of the 3D11 and 3G8 monoclonal antibodies to SHe peptide in a ELISA assay detected by either mouse IgG1 or mouse IgG2a specific secondary antibodies.

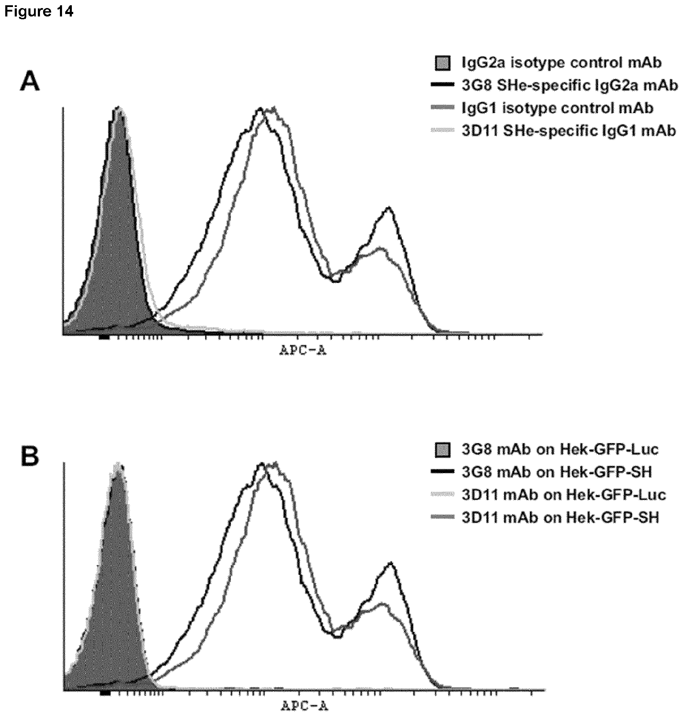

**Figure 14****:** 3D11 and 3G8 mAbs bind to the RSV SH ectodomain on living cells expressing the RSV SH protein on their cell surface. A, Flow cytometric analysis of the binding of 3D11 and 3G8 mAbs and respective isotype matched control antibodies to Hek293T cells expressing GFP and the RSV SH protein. B, Flow cytometric analysis of the binding of 3D11 and 3G8 mAbs to Hek293T cells expressing GFP in combination with either the RSV SH protein or a control protein (luciferase).

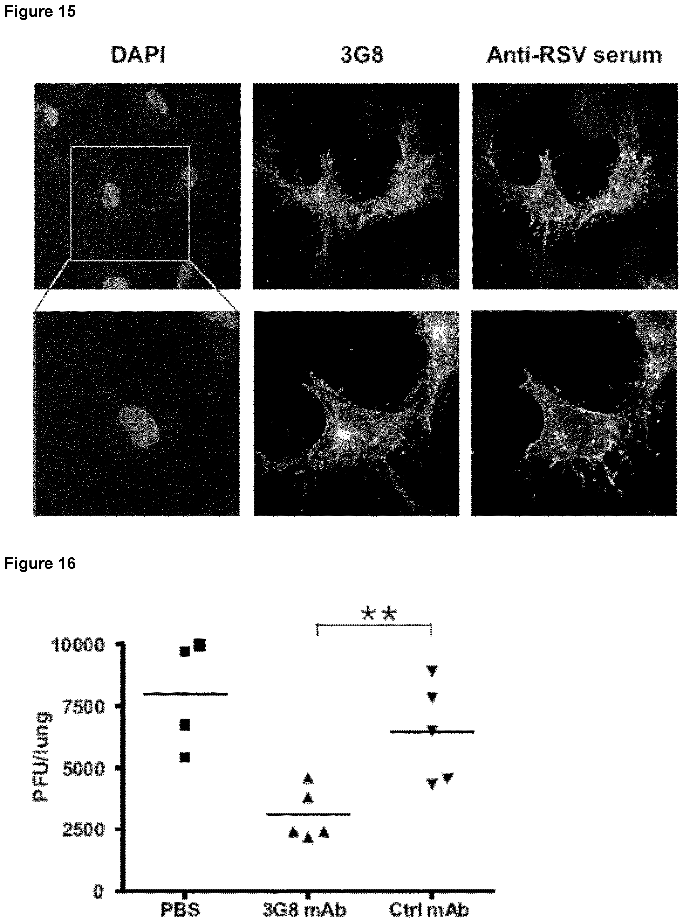

**Figure 15****:** Binding of 3D11 and 3G8 mAbs to the cell surface of RSV infected cells. Vero cells were infected with 0.5 MOI of RSV A2. Twenty hours after transfection, the cells were fixed, permeabilized and stained with 3D11 or 3G8 in combination with a polyclonal anti-RSV serum to identify the infected and non-infected cells. The upper panels represent an overview of the immunostaining (DAPI nuclear stain, 3D11 and polyclonal RSV serum), including infected and non-infected cells. The lower panels represent confocal images of an infected cell, indicated in the upper panel.
**Figure 16****:** Passive immunization with SHe specific monoclonal antibodies reduced RSV infection in mice. Balb/c mice were treated with PBS, SHe specific 3G8 mAb's or isotype control antibodies via intranasal administration one day before and one day after RSV challenge. Each symbol represents the lung virus titer of individual mice, four days after RSV challenge. (** p≤ 0.01).

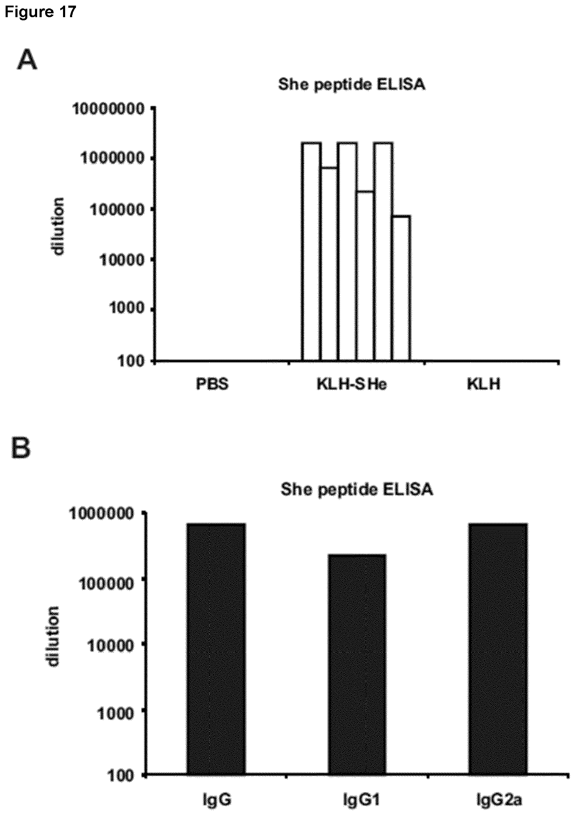

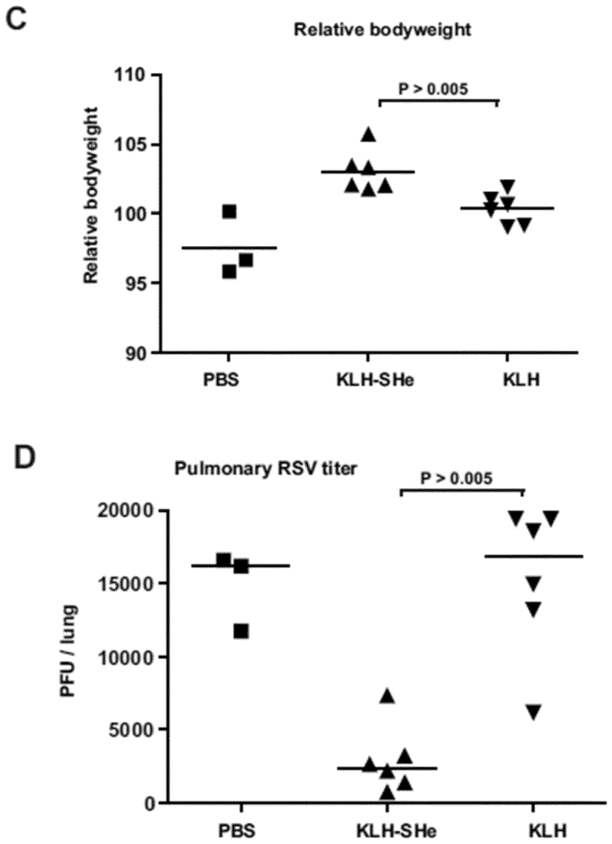

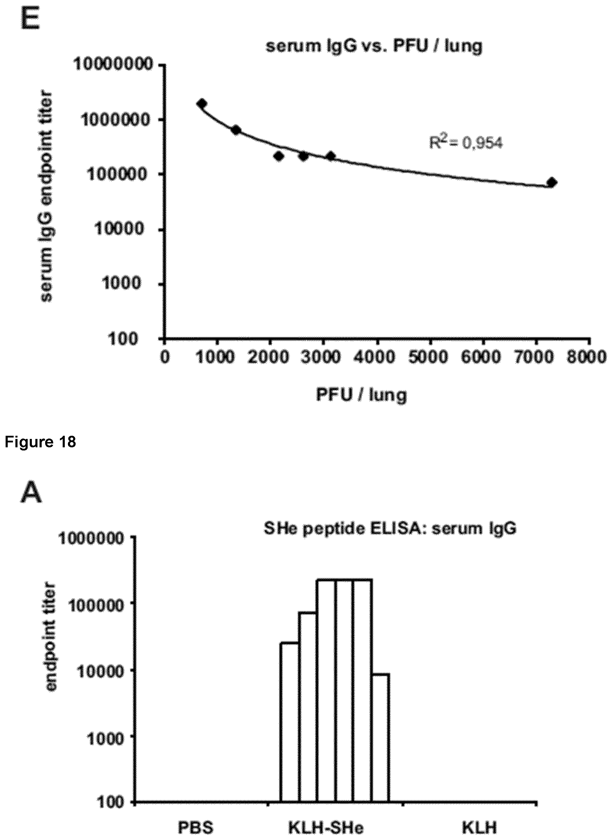

**Figure 17****:** Intraperitoneal vaccination of Balb/c mice with KLH-SHe in combination with Freund's Incomplete Adjuvant induces SHe-specific antibodies and reduces RSV replication. A, ELISA based determination of the She-specific IgG antibodies present in the sera of individual mice after the third immunization (boost 2) with the indicated vaccines. B, ELISA based determination of SHe-specific IgG, IgG1 and IgG2a antibodies present in the pooled sera of mice that were vaccinated with the KLH-SHe. C, KLH-SHe vaccination does not induce enhanced disease upon RSV infection. The graph shows the relative body weight of each mouse, calculated as the ratio between the weight on the day of sacrifice (five days after infection) and the weight on the day of viral infection, multiplied by 100. The difference in relative body weight between the KLH-SHe vaccinated and the KLH vaccinated mice is significant (p ≤ 0.005, Mann-Whitney U test). D, KLH-SHe vaccination impairs RSV replication. Five days after challenge with 10⁶ PFU RSV, the mice of the indicated groups were sacrificed and lung homogenates were prepared to determine the viral lung titer by plaque assay. The graph shows the number of plaque forming units per lung of each mouse. The detection limit of the plaque assay is 20 PFU per lung. The difference in RSV lung titer between the KLH-SHe vaccinated and the KLH vaccinated mice is significant (p ≤ 0.005, Mann-Whitney U test). E, For KLH-SHe vaccinated mice, high titers of SHe-specific serum antibodies strongly correlate with reduction of RSV replication. The graph shows for each KLH-SHe vaccinated mouse the titer of SHe-specific serum IgG antibodies and the number of PFU/lung that could be detected five days after infection. In the graph the best fitting curve (power) and its R2 (coefficient of determination) are shown.

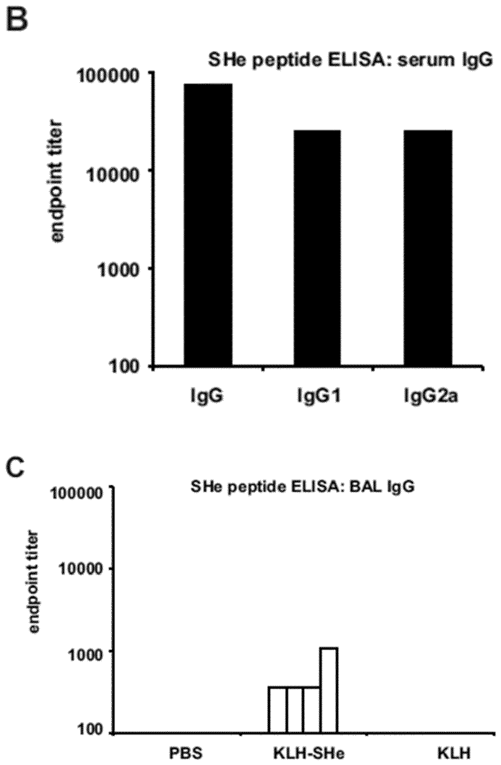

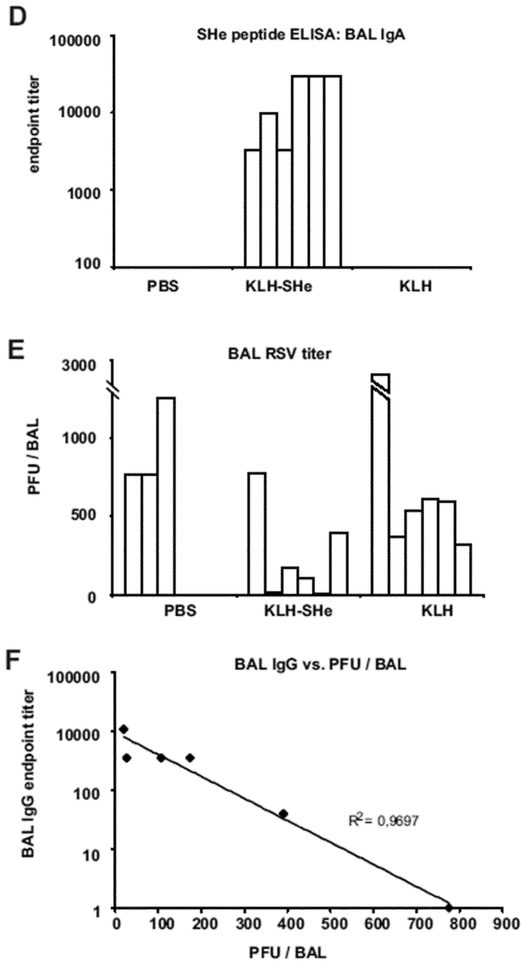

**Figure 18****:** Intranasal vaccination of Balb/c mice with KLH-SHe in combination with LTR192G induces SHe-specific antibodies and reduces RSV replication. A, ELISA based determination of the SHe-specific IgG antibodies present in the sera of individual mice after the third immunization (boost 2) with the indicated vaccines. B, ELISA based determination of the SHe specific IgG, IgG1 and IgG2a antibodies present in the pooled sera of mice that were vaccinated with KLH-SHe. C and D, ELISA based determination of the SHe-specific IgG and IgA antibodies present in the BAL fluid of individual mice that were vaccinated with the indicated vaccines and infected with RSV five day before the collection of BAL fluid. E, KLH- SHe vaccination impairs RSV replication. Five days after challenge with 10⁶ PFU RSV, the mice of the indicated groups were sacrificed to determine viral lung titer by plaque assay. The graph shows the number of plaque forming units per lung of each mouse. The detection limit of the plaque assay is 20 PFU per lung. The difference in RSV lung titer between the KLH-SHe vaccinated and the KLH vaccinated mice is significant (p ≤ 0.05, Mann-Whitney U test). F, For KLH-SHe vaccinated mice, high titers of SHe-specific IgG antibodies present in the BAL fluid strongly correlate with reduction of RSV replication. The graphs shows for each KLH-SHe vaccinated mouse the titer of SHe-specific BAL IgG antibodies and the number of PFU/lung
   that could be detected five days after infection. In the graph the best fitting curve and its R2 (coefficient of determination) are shown.

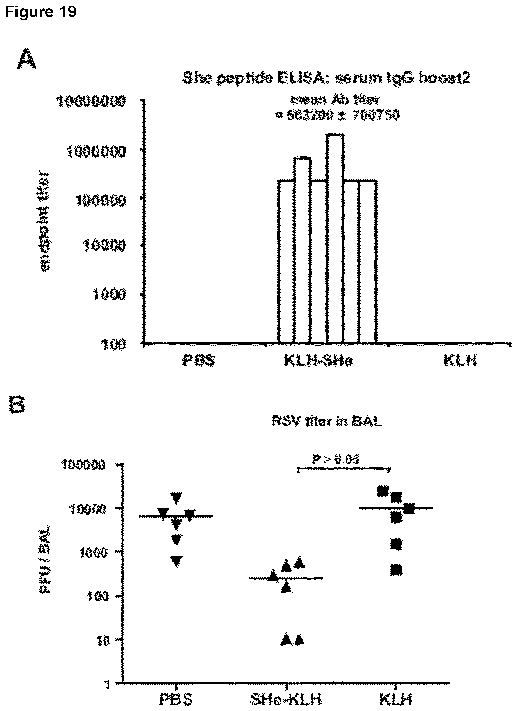

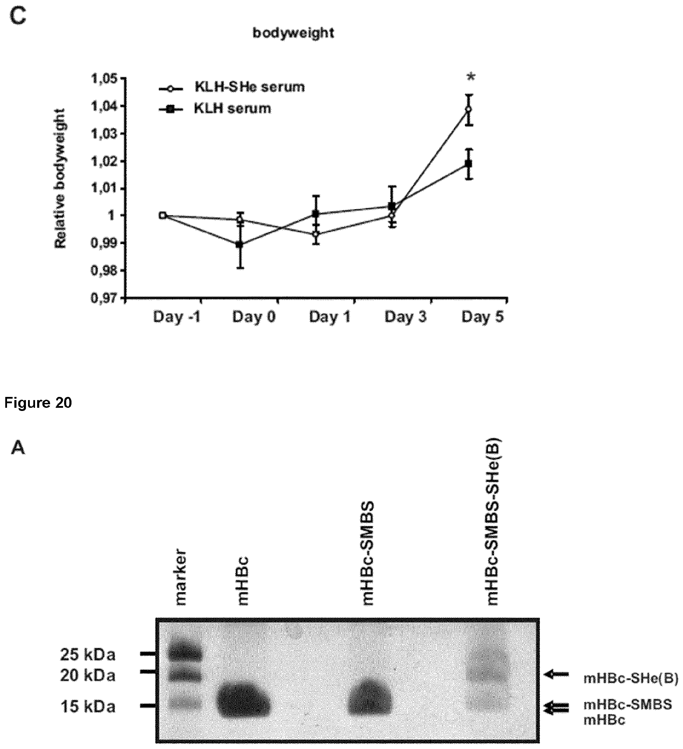

**Figure 19****:** Passive immunization with KLH-SHe immune serum reduces RSV infection in mice. A, ELISA based determination of the SHe-specific IgG antibodies present in the sera of individual mice after the third immunization (boost 2) with the indicated vaccines. B, Passive immunization with KLH-SHe immune serum reduces RSV infection in mice. Serum from KLH- SHe or KLH vaccinated mice or PBS were administrated intranasally to mice one day before and one day after RSV challenge. Five days after challenge with 10⁶ PFU RSV, the mice of the indicated groups were sacrificed and lung homogenates were prepared to determine the viral lung titer by plaque assay. The graph shows the number of plaque forming units per lung of each mouse. The detection limit of the plaque assay is 20 PFU per lung. The difference in RSV lung titer between the KLH-SHe vaccinated and the KLH vaccinated mice is significant (p ≤ 0.05, Mann-Whitney U test). C, Passive immunization with KLH-SHe serum does not induce enhanced disease upon RSV infection. The graph shows the mean +/- SEM relative body weight of each mouse, calculated as the ratio between the weight at a specific day and the weight at the day of the first passive immunization, multiplied by 100. The difference in relative body weight between the mice that were treated with KLH-SHe serum and the mice that were treated with KLH serum is significant (p ≤ 0.005, Mann-Whitney U test).
**Figure 20****:** Chemical linkage of SHeB peptides to the immunodominant loops of mHBc virus like particles. Coomassie blue stained SDS-PAGE analysis of mHBc VLPs, mHBc VLPs linked to the SMBS heterobifunctional crosslinker (mHBc-SMBS) and purified mHBc-SMBS VLPs with chemically linked SHeB peptides (mHBc-SHeB).

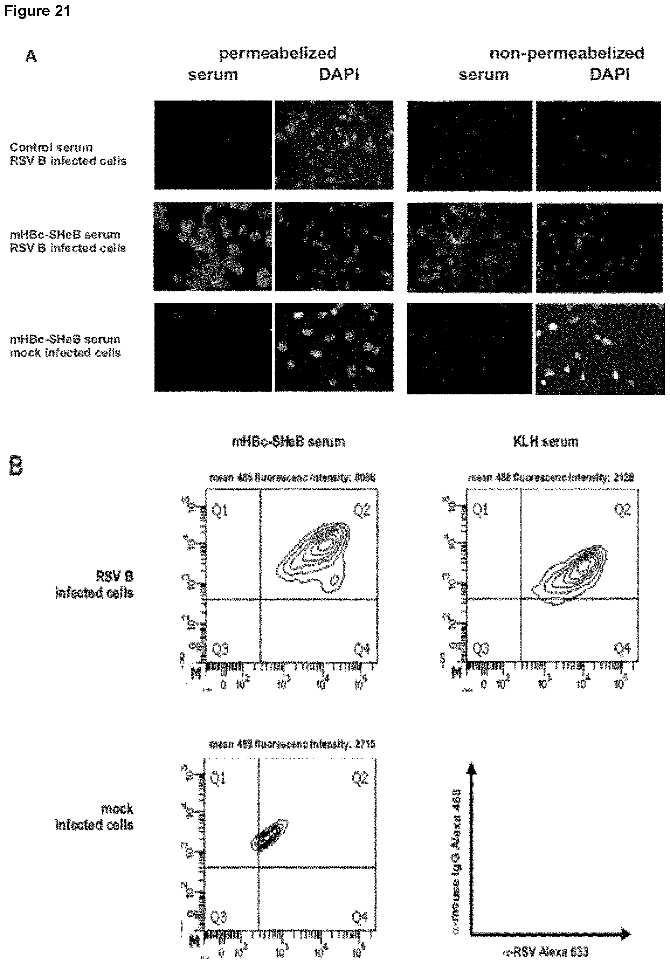

**Figure 21****:** Binding of Serum of mHBc-SHeB vaccinated mice to the surface of RSV B infected cells. Vero cells were infected with a RSV B clinical isolate or mock infected. Seventy-two hours after infection, the cells were fixed and either permeabilized or not permeabilized. Infected and mock infected cells were stained with Serum of a mHBc-SHeB vaccinated mouse or with serum of KLH vaccinated mice, as indicated. Binding of mHBc-B or KLH serum antibodies to the cells was analyzed by using Alexa488 linked anti-mouse IgG antibodies. A, For microscopic analysis the cells were also stained with the nuclear dye DAPI. B, For flowcytometric analysis the non permealized cells were also stained with a goat anti-RSV serum to identify the RSV B infected cells. Binding of goat anti-RSV serum antibodies to the cells was determined by using Alexa633 linked anti-goat IgG antibodies. The graphs represent Alexa488 intensity / Alexa633 intensity contour plots of the indicated cells.

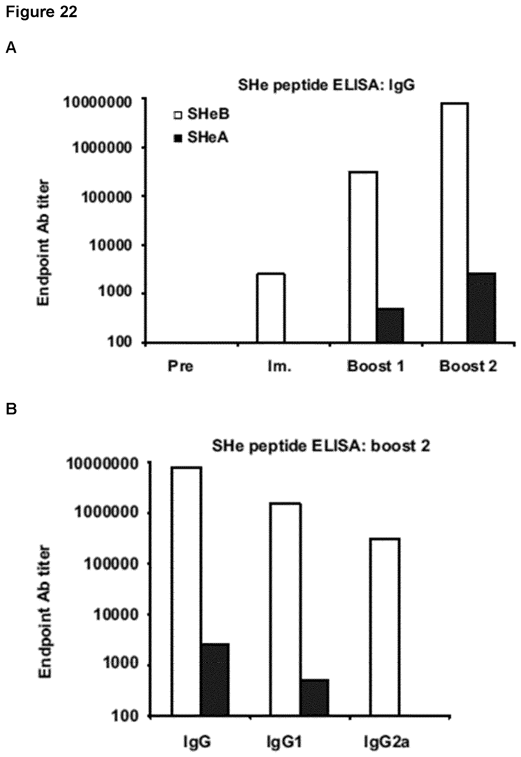

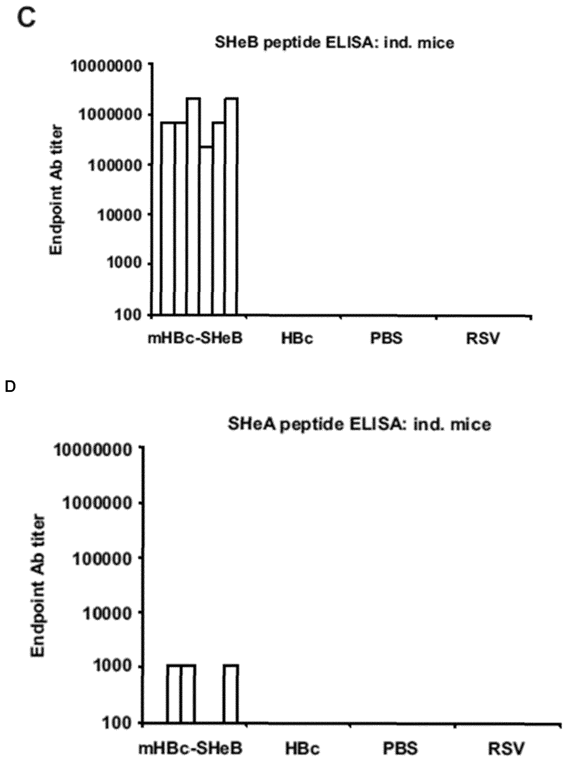

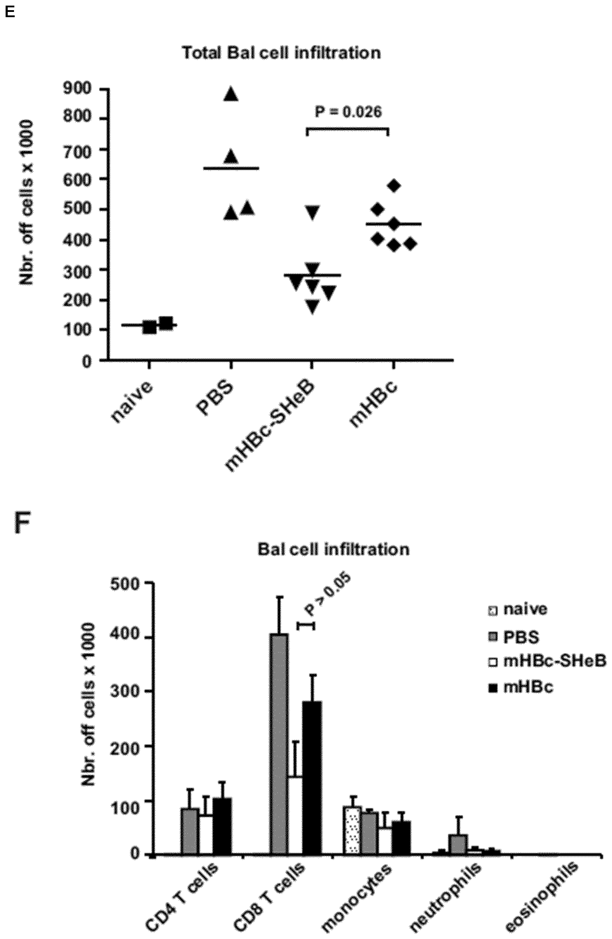

**Figure 22****:** Vaccination with mHBc-SHeB induces SHeB specific antibodies and reduces RSV B induced pulmonary inflammation. A, ELISA based determination of the SHeB- and SHeA- specific IgG antibodies present in the pooled sera of mice after the first (im.), the second (boost 1) and third mHBc-SHeB immunization (boost 2). B, ELISA based determination of the SHe-specific IgG, IgG1 and IgG2a antibodies present in the pooled sera of mice that were vaccinated with KLH-SHe. C and D, ELISA based determination of SHeB-(C) and SHeA- specific (D) IgG antibodies present in the sera of individual mice that were vaccinated with the indicated vaccines. E, The total number of cells present in the BAL fluids of RSV infected mice that had been vaccinated with the indicated vaccines. There are significantly less cells present in the BAL fluid of mice that had been vaccinated with mHBc-SHe compared to BAL fluid of mice that had been vaccinated with mHBc. (p ≤ 0.05, Mann-Whitney U test). F, The number of CD4+ T cells, CD8+ T cells, monocytes, neutrophils and eosinophils present in the BAL fluids. There are significantly less CD8+ T cells present in the BAL fluid of mice that had been vaccinated with mHBc-SHe compared to the BAL fluid of mice that had been vaccinated with mHBc. (p ≤ 0.05, Mann-Whitney U test).

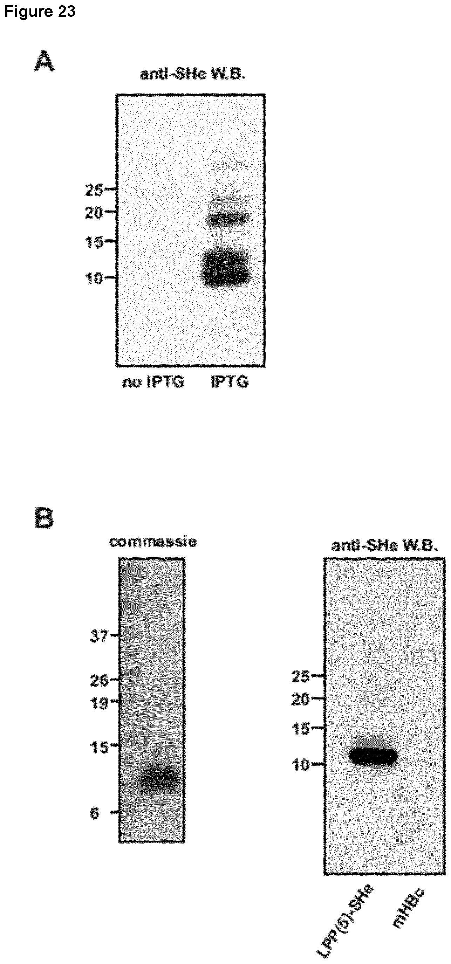

**Figure 23****:** Expression and purification of the LPP₍₅₎-SHe protein. A, Expression of the LPP₍₅₎- SHe protein. pLH36-HisDEVD-LPP₍₅₎-SHe transformed *E. coli* cells were either stimulated with 1 mM 1-thio-p-d-galactopryanoside (IPTG) or not. Four hours later crude extracts were prepared by sonication followed by centrifugation (13 000 x g, 30 min, 4°C). The supernatant was analyzed by SDS-PAGE and Western blotting using the SHe-specific 3G8 monoclonal antibody. B, Analysis of purified LPP₍₅₎-SHe protein. After purification, the LPP₍₅₎-SHe protein was analyzed by SDS-PAGE, commassie blue staining (left) and Western blot (right) analysis using the SHe-specific 3G8 monoclonal antibody.

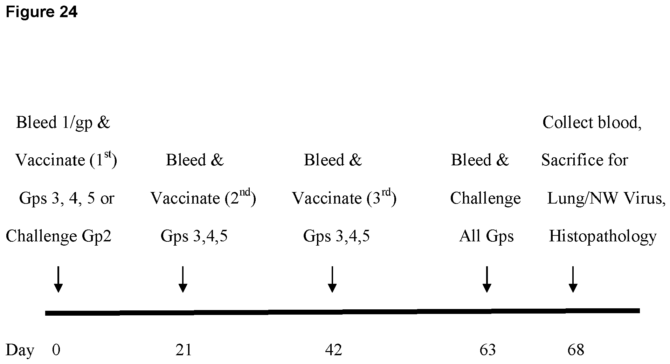

**Figure 24****:** schedule of the vaccination of the cotton rats. Group numbers refer to:
   Group 1 : 6 Cotton rats (CR) no vaccine and challenged with RSV on day +63 (infection control)
   Group 2: 6 CR inoculated intranasally with RSV-Tracy at 2.04 x 105 PFU/CR on Day 0
   Group 3: 6 CR each, vaccinated intraperitoneal^ (IP) with KLH-SHe + IFA
   Group 4: 6 CR each, vaccinated intraperitoneal^ (IP) with KLH + IFA (vehicle control)
   Group 5: 6 CR each, vaccinated intramuscularly (IM) with 1:10 formalin-inactivated (FI) RSV- Bernett grown in Vero cells (positive control for immune exacerbation upon challenge)

### EXAMPLES

### Materials and methods to the examples

### Cloning and plasmid construction.

*Construction of the pLT32 Flag-COMPcc-SHe expression plasmid.* A plasmid, containing the coding sequence of Flag-COMPcc-SHe (Figure 1.B) was ordered at Genscript (SEQ ID N° 31). The Flag-COMPcc-SHe coding sequence was ligated as a Ndel/Notl fragment in a Ndel/Notl opened pLT32H bacterial expression vector (Mertens et al., 1995)

*Construction of the pCAGGS-Etag-SH expression vector.* Total RNA of RSV A2 infected Hep-2 cells was prepared using the high pure RNA tissue kit (Roche, Mannheim) according to the manufacturer's instructions. After cDNA synthesis the RSV A2 SH coding sequence was amplified using the following forward and reverse primers (5'ATAAGAAAGCGGCCGCTATGGAAAATACATCCATAACAATAG3'; 5 AAGATCTCTATGTGTTGACTCGAGCTCTTGGTAACTCAAA3'). The PCR product was digested with Notl and Bglll and ligated in a Notl/Bglll opened pCAGGS-PTB-Etag expression vector (Cornells et al., 2005). The resulting vector pLT32-Flag-COMPcc-SHe was deposed under the Budapest treaty at BCCM (BCCM/LMBP: Technologiepark 927, 9052 Zwijnaarde, Belgium) under deposition number LMBP 6817 on 8 November 2010.

*The construction of the pCAGGS-Luc expression vector* was described earlier (Schepens et al., 2005; referred as pCAGGS-HIF-RLuc)

*Construction of the pLT32 mHBc expression vector.* The coding sequence of mHBc, as described earlier by Jegerlhener et. al. as part of the "ab1" plasmid, was ordered at Geneart (SEQ ID N° 32) (De Filette et al., 2005; Jegerlehner et al., 2002). This coding sequence was cloned as a Ndel/Notl fragment in a Ndel/Notl opened pLT32H bacterial expression vector.

*Construction of the pLT32 SHe-tGCN4-Flag expression vector.* To construct pLT32 SHe-tGCN4 the SHe coding sequence was fused to the tGCN4-Flag coding sequence by fusion per. The SHe fragment for fusion per was amplified using the primers: 5 GAATTCCATATGAACAAGTTATGTGAGTACAACG3' and 5 ATTTGTTTTAAACCTCCTGTATTTACTCGTGCCCGAGGCAA3' and a template plasmid that was ordered at Geneart (SEQ ID N° 33) and which contains the coding sequence of the RSV A2 SH ectodomain (NKLCEYNVFHNKTFELPRARVNT) (SEQ ID N° 40). The GCN4 fragment for fusion PCR was amplified using the primers 5 CCAAGCTTCTAACATTGAGATTCCCGAGATTGAGA3' and 5TATTAACCCTCACTAAAGGGAAGG3' and a template plasmid which contains the tGCN4 coding sequence, C-terminally fused to the coding sequence of 3 successive Flag-tag
sequences (SEQ ID N° 34; De Filette et al., 2008). The two PCR fragments were fused using the primers: 5'GGAATTCCATATGAACAAGTTATGTGAGTACAACG3' and 5' TATTA ACC CTC ACTA A AG G G A AG G 3' . This fusion PCR product was cloned as a Ndel/Hindlll fragment in a Ndel/Hindlll opened pLT32H bacterial expression vector. The resulting pLT32 SHe-tGCN4-Flag was deposed under the Budapest treaty at BCCM (BCCM/LMBP: Technologiepark 927, 9052 Zwijnaarde, Belgium) under deposition number LMBP 6818 on 8 November 2010.

The construction of the PLT32 M2e-tGCN4 expression vector was described earlier (De Filette et al., 2008).

*Construction of the pLH36-HisDEVD-LPP(5)-SHe expression plasmid.* A plasmid containing the coding sequence of the LPP(5) tryptophan-zipper fused to the coding sequence of the SH ectodomain separated by the coding sequence of a GlyGly linker was ordered at Genscript. This coding sequence was amplified using the following forward and reverse primers (5 'GCGAAATGGGATCAGTGGAGCAGC-3'; 5'AATATAGGATCCCTAGGTCGCCCAGTTATCCCAGCG-3'), phosphorylated and digested with BamH\. The pLH36-HisDEVD-LPP-SHe was constructed by a tree-point ligation using the described PCR fragment, BamHVPst\ digested pLT32 plasmid fragment and EcoRV/Pst\ digested pLH36 fragment. The sequence of the constructed pLH36-HisDEVD-LPP₍₅₎-SHe plasmid is displayed in SEQ ID N° 49.

### Expression and purification of SHe-tGCN4, M2e-tGCN4, Fiag-COMPcc-SHe, mHBc and LPP₍₅₎-SHe.

A 30-ml preculture of pLT32SHe-tGCN4 transformed E. coli was grown at 28 °C in Luria broth and used to inoculate 1 liter of fresh medium. At an A600 of 0.6-0.8, the cells were treated with 1 mm isopropyl 1-thio^-d-galactopyranoside, incubated for another 4 h, and then collected by centrifugation (6000 <χ> g, 20 min, 4 °C). The bacterial pellet was resuspended in 20 ml Tris-HCI buffer (50mM Tris-Hcl, 50mM NaCI and 1mM EDTA), pH 8, and sonicated. Bacterial debris was pelleted by centrifugation (20,000 <χ> g, 1 h, 4 °C). The supernatant was applied to a DEAE Sepharose column pre-equilibrated with Tris-HCI buffer containing 50 mM NaCI (buffer A). After washing the bound proteins were eluted by a two-step gradient going from 0-40% buffer B (50 mM Tris-Hcl, 1 M NaCI) and 40-100% buffer B. Fractions containing SHe-tGCN4 were pooled, adjusted to 25% ammonium sulfate saturation, and applied to a phenyl-Sepharose column pre-equilibrated with 25% ammonium sulfate, 50 mm Tris-HCI, pH 8. Bound proteins were eluted with a two-step gradient. The two-step elution was performed with 0-40 and 40-100% 50 mM Tris-HCI buffer, pH 8 (buffer A). The fractions containing SHe- tGCN4 were loaded on a Superdex 75 column. Gel filtration was performed in phosphate-buffered saline (PBS), and the fractions containing SHe-tGCN4 were pooled and stored at - 70 °C.

Expression and purification of flag-COMPcc-SHe was identical to SHe-tGCN4 apart from the use of a Q Sepharose column for anion exchange chromatography instead of a DEAE Sepharose column.

The expression and purification of M2e-tGCN4 was described before (De Filette et al., 2008) Expression and purification of mHBc was identical to SHe-tGCN4 apart from the use of a Sephacryl S400 column for gelfiltration chromatography instead of Superdex 75 column.

*Expression and purification of LPP₍₅₎-SHe.* A 30-ml preculture of pLH36-HisDEVD-LPP₍₅₎-SHe transformed *E. coli* cells was grown at 28 °C in Luria broth with ampiciline and used to inoculate 3 liter of fresh medium. At an A₆₀₀ of 0.6-0.8, the cells were treated with 1 mM isopropyl 1-thio-β-d-galactopyranoside, incubated for another 4 h, and then collected by centrifugation (6000 x g, 20 min, 4 °C). The bacterial pellet was resuspended in 300 ml buffer containing 20mM NaH₂P0₄/Na₂HPO₄, 300 mM NaCI and 5mM imidazole, pH 7,5 and sonicated. Bacterial debris was pelleted by centrifugation (20,000 x g, 1 h, 4 °C). The supernatant was loaded on a Nickel-Sepharose column pre-equilibrated with buffer containing 5 mM Imidazole. After washing, the bound proteins were eluted by a step-wise (50 mM, 100 mM, 200 mM and 400 mM) imidazole gradient. Fractions containing LPP₍₅₎-SHe were pooled, desalted and further purified on a Q-sepharose column. The sample was applied to a DEAE Sepharose column pre-equilibrated with Tris-HCI buffer containing 50 mM NaCI (buffer A). After washing the bound proteins were eluted by a two-step gradient going from 0-40% buffer B (50 mM Tris-Hcl, 1 M NaCI) and 40-100% buffer. The fractions containing LPP₍₅₎-SHe were loaded on a Superdex 75 column. Gel filtration was performed in phosphate-buffered saline (PBS), and the fractions containing LPP₍₅₎-SHe

### Adjuvants

A detoxified mutant of heat-labile E. coli enterotoxin, LTR192G, was used for intranasal (i.n.) administration; this preparation was generously provided by Dr. J. Clements (Department of Microbiology and Immunology, Tulane University Medical Center, New Orleans, LA, USA) (Norton et al., 2010).

### Chemical linking and characterization of She-HBc partikels.

SHe(cc4s) a chemically synthesized, HPLC-purified SHe peptide in which the naturally occurring cystein was replaced by a serine and to which a cystein was added at the N-terminus was ordered at Pepscan (Pepscan, Lelystad). The SHe(cc4s) peptide was via its N-terminal cysteine residue fused to a Lysine in the immunodominant loop of mHBc on the surface of HBc VLP's by chemical linkage using the heterobifuctional sulfo-MBS (Pierce), according to the manufacturer's instructions. In short 400µg mHBc, dissolved in 200 µl PBS, was incubated with Sulfo-MBS (at a final concentration of 1 mg/ml) for one hour. After removal of unbound Sulfo-MBS molecules by size exclusion chromatography, sulfo-MBS linked mHBc VLPs were diluted in 2ml H20. Subsequently 100 µl SHe(cc4s) peptide (disoleved in 100 ml PBS) was added and incubated for one hour at room temperature to allow cross-linking of the peptide to the mHBc VLPs. Finally, free SHe(cc4s) peptide was removed by size exclusion chromatography. The purity and cross-linking efficacy was tested via SDS-PAGE followed by coomassie staining.

### Cells

Hep-2 cells (ATCC, CCL-23), Vero cells (ATCC, CCL-81), HEK293T cells (a gift from Dr M. Hall) and A549 cells ATCC, CCL-185) were grown in DMEM medium supplemented with 10% heat-inactivated fetal calf serum (FCS), 1 % penicillin, 1 % streptomycin, 2mM L-glutamine, non- essential amino acids (Invitrogen, Carlsbad, Carlifornia), and 1 mM sodium pyruvate.

### Mice and viruses.

Specific pathogen-free, female BALB/c mice were obtained from Charles River (Charles River Wiga, Sulzfeld, Germany). The animals were housed in a temperature-controlled environment with 12-h light/dark cycles; food and water were delivered ad libitum. Mice were immunized at 8 weeks of age after 1-week adaptation in the animal room.

The animal facility operates under the Flemish Government License Number LA1400091. All experiments were done under conditions specified by law (European Directive and Belgian Royal Decree of November 14, 1993) and authorized by the Institutional Ethical Committee on Experimental Animals

RSV A2 an A subtype of RSV, (ATCC, Rockville) was propagated by infecting monolayers of Vero cells, with 0.1 MOI in the presence growth medium containing 1 % FCS. Five to seven days after infection the cells and growth medium were collected, pooled and clarified by centrifugation (450 x g). To concentrate the virus, the clarified supernatant was incubated for 4 hours at 4°C in the presence of 10% polyethylene glycol (PEG6000). After centrifugation (30 minutes at 3000 x g), the pellet was resuspended in Hank's balanced solt solution (HBSS), containing 20% sucrose, aliquoted and stored at -80°C.

### Intranasal immunizations and infections.

For intranasal immunization or infection the mice were slightly anesthetized by isoflurane. The final volume used for administration of vaccine + adjuvant or virus was 50 µl (25 µl per nostril).

Vaccines + adjuvant were formulated in PBS whereas the viral inoculum was formulated in HBSS.

### Determination of lung viral titer by Plaque assay.

Three or four days postchallenge the mice were sacrificed. The mouse lungs were removed aseptically and homogenized with a Heidolph RZR 2020 homogenizer for 30 seconds in 1 ml HBSS containing 10% sucrose. Lung homogenates were subsequently cleared by centrifugation at 4°C and used for virus titration on Hep-2 cells. Monolayers of Hep-2 cells were infected with 50 µl of serial 1:3 dilutions of the lung homogenates in a 96-well plate in serum-free OPTIMEM medium (Invitrogen) supplemented with penicillin and streptomycin. Four hours later the cells were washed twice with DEMEM medium containing 2% FCS and incubated for five days at 37°C in 50µl overlay medium (completed DEMEM medium containing 1% FCS, 0.5% agarose). The cells were fixed by adding 50µl of a 4% paraformaldehyde solution on top of the agarose overlay. After overnight fixation at 4°C the overlay medium and paraformaldehyde solution were removed, the cells were washed twice with PBS and blocked with PBS containing 1% BSA (PBS/BSA). Subsequently, polyclonal goat anti RSV serum (AB1128, Chemicon International) was added (1/4000). After washing three times with PBS/BSA, the cells were incubated with hrp-conjugated anti goat IgG antibodies (SC2020, Santa Cruz) for 30 minutes. Non-binding antibodies were removed by washing four times with PBS/BSA containing 0.01% Triton-X100 and once with PBS. Finally, the plaques were visualized by the use of TrueBlue peroxidase substrate (KPL, Gaithersburg). The plaques of different dilutions were counted and for each dilution the number of PFU per lung (1 ml) was calculated as: number of plaques present in the dilution x the dilution x 20 (= 1000 µl total supernatant volume / 50 µl of the volume of supernatant used to infect the first well of the dilution series). The number of PFU/lung was than calculated as the average number of PFU/lung calculated for the different dilutions. As each supernatant of the homogenized lungs was tested in duplicate, the final number of PFU/lung was calculated as the average of these duplicates.

### Determination of lung viral titer by qRT-PCR.

To determine the lung RSV load by qRT-PCR, lung homogenates were prepared and clarified as described above. Total RNA form these lung homogenates was prepared by the use of the High Pure RNA tissue Kit (Roche, Mannheim) according to the manufacturer's instructions. cDNA was prepared by the use of hexamer primers and the Transcriptor First Strand cDNA synthesis kit (Roche, Mannheim). The relative levels of genomic RSV M cDNA were determined by the use of by qRT-PCR using primers specific for the genomic RNA of the RSV A2 M-gene (5TCACGAAGGCTCCACATACA3' and 5 CAGGGTCATCGTCTTTTTC3') and a
nucleotide probe (#150 Universal Probe Library, Roche) labeled with fluorescein (FAM) at the 5'-end and with a dark quencher dye near the 3'- end. The relative amount of GADPH mRNA was determined by qRT-PCR using primers specific for mouse GADPH (5TGAAGCAGGCATCTGAGGG3' and 5 GAAGGTGGAAGAGTGGGAG3' and LightCycler 480 SYBR Green I Maseter Mix (Roche). The relative amount of genomic RSV RNA per lung homogenate was calculated as the ratio between the relative amount of RSV M-gene RNA and the relative amount of mouse GADPH mRNA.

### Peptide ELISA

Two weeks after each immunization, blood samples were collected from the lateral tail vein. The final bleeding was performed by cardiac puncture of animals anesthetized with avertin. Blood was allowed to clot for 30 min at 37 °C, and serum was obtained by taking the supernatant from two subsequent centrifugations.

Serum antibody titers were determined by ELISA using pooled sera from the group. To determine M2e or SHe-specific antibody titers, microtiter plates (type II F96 MaxiSorp, Nunc) were coated with respectively 50 µl of a 2 µg/ml M2e-peptide solution or 2 µg/ml SHe-peptide solution in 50 mM sodium bicarbonate buffer, pH 9.7, and incubated overnight at 37 °C. After washing, the plates were blocked for 1 h with 200 µl of 1 % BSA in PBS. After 1-h incubation, the plates were washed again. A series of 1/3 dilutions of the different serum samples, starting with a 1/100 dilution, were loaded on the peptide-coated plates. The bound antibodies were detected with a peroxidase-labeled antibody directed against mouse isotypes IgG1 or IgG2a (Southern Biotechnology Associates, Inc., Birmingham, AL, USA) and diluted 1/6000 in PBS + 1% BSA + 0.05% Tween 20. After washing, the microtiter plates were incubated for 5 min with TMB substrate (Tetramethylbenzidine, Sigma-Aldrich). The reaction was stopped by addition of an equal volume 1 M H3PO4 and the absorbance at 450 nm was measured. Endpoint titers are defined as the highest dilution producing an O.D. value twice that of background (pre-immune serum).

### Flow cytometric analysis.

Hek293T cells were transfected with the indicated expression vectors. Twenty-four hours later the cells were detached using enzyme free dissociation buffer (Invitrogen, Carslbad, California), washed once with PBS and incubated for one hour in PBS containing 1% BSA (PBS/BSA). Subsequently the cells were incubated with the indicated serum or antibodies at the indicated concentrations. One hour later the cells were washed 3 times with PBS/BSA and incubated with the anti-mouse IgG alexa 633 secondary antibodies for 30 minutes. After washing the cells four times with PBS/BSA and once with PBS, the cells were analyzed using a Becton Dickinson LSR II flowcytometer. Single GFP expressing cells were selected based on
the peak surface of the sideward scatter signal, the peak surface and peak height of the forward scatter signal and the peak surface of the green fluoresence signal. Finally of these GFP positive single cells, the alexa 633 fluoresence signal was measured.

### Immunostaining.

Vero cells were either mock infected or infected with 0,5 MOI of RSV A2 in the presence of serum free medium. Four hours later the free virus was washed away and the cells were incubated in growth medium containing 1% FCS. Sixteen hours later the cells were washed once with PBS and fixed with 2% paraformaldehyde for 20 minutes. Subsequently the cells were washed twice with PBS and permeabilized with 0,2% Triton-X100 detergent for 5 minutes. After washing once with PBS the cells were blocked in PBS/BSA. One hour later She specific 3G8 monoclonal antibody or isotype control antibody was added at a final concentration of 5µg/ml. After washing the cells twice with PBS/BSA polyclonal anti RSV goat serum was added. One hour later, the cells were washed three times with PBS/BSA. The binding of the indicated antibodies to the cells was analyzed by the use of anti-mouse and anti- goat IgG antibodies labeled with respectively alexa 488 and alexa 568 fluorescent dyes. Confocal images of the stained cells were recorded with a Zeiss confocal microscope.

### Generation of SHe mAb producing hybridomas

Stable hybridomas cells producing SHe-specific monoclonal antibodies (mAb) were generated by hybridoma technology (Kohler and Milstein 1975). Briefly SHe-specific hybridomas were derived from individual mice that were immunized i.p. three times at three weeks intervals with 10 µg of SHe-tGCN4 vaccine adjuvanted with allhydrogel (Brenntag Biosector). Three days before fusion, mice were boosted an additional time with the same formulation and splenocytes were isolated then fused to SP2/0-Ag14 myeloma cells in the presence of PEG 1500 (Roche Diagnostics GmbH, Germany). Fused cells were grown in RPMI 1640 medium supplemented with 10% Fetal bovine serum, 10% BM condimed HI (Roche Diagnostics GmbH, Germany), 2 mM L-glutamine, and 24 µM beta-mercaptoethanol and 1x HAT supplement (Invitrogen, Carlsbad, Carlifornia). Hybrids secreting SHe-specific antibodies were identified by SHe peptide Elisa screening and monoclonal antibodies producing hybrids were obtained after two rounds of sub-cloning by limiting dilution procedure. Monoclonal antibodies were purified on a protein A-Sepharose column (electrical engineering biosciences).

The resulting hybridomas were deposed under the Budapest treaty at BCCM (BCCM/LMBP: Technologiepark 927, 9052 Zwijnaarde, Belgium) under deposit numbers LMBP 7795CB for 3G8 on 8 November 2010 and LMBP 7796CB for 3D11 on 10 November 2010, respectively.

### Example 1: Design, expression and purification of Flag-COMPcc-She

The SH protein is expressed at the surface of RSV virions and the plasma membrane of RSV infected cells as a pentamer. The pentameric organization of SH is organized by the SH transmembrane domain which oligomerizes as a coiled coil of 5 parallel alpha-helices. In order to present the C-terminal SH ectodomain (SHe) of RSV A as a pentamer that mimics its natural conformation, SHe was genetically fused to the short pentameric coiled coil domain of the rat cartilage oligomeric matrix protein (COMPcc) which is, also composed of 5 parallel alphahelices (Malashkevich et al., 1996; Figure 1). A Flag-tag was fused to the N-terminus of COMP, rendering Flag-COMPcc-SHe. Flag-COMPcc-SHe was cloned in an pLT-32 (Mertens et al., 1995)_expression vector, expressed in *E. coli* and purified. Gel filtration analysis revealed that Flag-COMPcc-SHe eluted as a 55-60 kDa complex, indicating that the 11 kDa Flag-COMPcc-SHe proteins do indeed oligomerize into pentamers (Figure 2).

### Example 2: Flag-COMPcc-SHe vaccination induces SHe specific antibodies and protection against RSV infections

To test if vaccination with Flag-COMPcc-SHe could evoke protection against RSV infection we used a BALB/c mouse RSV infection model. BALB/c mice were immunized three times intranasally with 25 µg of Flag-COMPcc-SHe in combination with 1 µg E. coli heat-labile enterotoxin LTR192G adjuvant. PBS and the Influenza A M2 ectodomain fused to a tetrameric GNC4 scaffold (M2e-tGNC4) (De Filette et al., 2008) were used as negative controls. Immunizations were performed every fortnight. A single RSV infection (5X105 PFU) was used as positive control. Between the first and the second week after each immunization blood was collected to investigate the induction of SHe-specific IgG antibodies. The presence of SHe- specific antibodies was first tested by SHe peptide ELISA. M2e peptide ELISA was used as negative control. Figure 3 demonstrates that SHe peptide-specific IgG antibodies are induced and boosted after respectively the second and third immunization with Flag-COMPcc-SHe. Three successive Flag-COMPcc-SHe/LTR192G immunizations resulted in high levels of lgG2a SHe-specific antibodies but only low levels of IgG1 SHe-specific antibodies, indicating a Th1 oriented/driven immune response. No SHe spefic IgG antibodies could be detected in PBS or M2e-tGCN4/LTR192G vaccinated mice (Figure 3A, B and C). As expected no M2e specific antibodies could be detected in the sera of Flag-COMPcc-SHe/LTR192G or PBS vaccinated mice data. Mice that were immunized with M2e-tGCN4 accumulated a high titer of M2e- specific lgG2a antibodies, in accordance with previous results (De Filette et al., 2008).

Next, we investigated if SHe specific antibodies present in the Flag-COMPcc-SHe immune serum could bind to cells expressing the RSV-SH protein at their surface by flow cytometry. HEK-293T cells were transfected with a GFP expression vector, in combination with either a SH expression vector (pCAGGS-Etag-SH) or a Luciferase expression vector (pCAGGS-Luc)
as negative control. Twenty-four hours after transfection the cells were detached, stained with different dilutions of Flag-COMPcc-SHe or M2e-tGCN4 immune serum and analyzed by flow cytometry. Figure 4 illustrates that in contrast to M2e-tGCN4 immune serum, serum from Flag- COMPcc-SHe vaccinated mice specifically binds SH protein expressed at the surface of living cells.

To test if Flag-COMPcc-SHe/LTR192G vaccination can elicit protection against RSV infection, the mice were challenged with 1X10⁶ PFU RSV A2, nine weeks after the last immunization. Four days after infection the mice were sacrificed to determine the viral lung titer by plaque assay. Figure 5 illustrates that compared to PBS and M2e-tGCN4 vaccinated mice, vaccination with Flag-COMPcc-SHe lowered RSV replication. No virus was detected in the mouse that was infected with living RSV before challenge.

It is well known that vaccination with formalin inactivated virus or the RSV G protein can induce enhancement of disease upon infection, resulting in significant morbidity, by the induction of an unbalanced Th2 immune response (Prince et al., 1986). To test if also Flag-COMPcc-SHe vaccination might induce enhancement of disease, we monitored the body weight before and after RSV challenge (Figure 6). No weight loss was observed in any of the mouse groups after RSV challenge. This strongly suggests that Flag-COMPcc-SHe vaccination does not result in enhancement of disease upon RSV infection.

### Example 3: Design, construction and purification of mHBc-SHe

The Hepatitis B virus core protein (HBc) Virus like particle (VLP) can present antigens as a dense array. In this way HBc-VLPs can induce a strong humoral immune response towards the presented antigen (Boisgerault et al., 2002). Therefore as an alternative to presenting SHe as a pentamer, the SH ectodomain was presented in the immunodominant region loop of mHBc-VLPs. HBc-SHe-VLPs were obtained by chemical linkage of SHe peptides to mHBc, a mutant of HBc in which a lysine was introduced in the top of the HBc immunodominant region (De Filette et al., 2005). To enable chemical linking a cysteine residue was added to the N- terminus of SHe. In addition the cysteine residue, present at position 4 of the SHe peptide, was replaced by a serine residue. This peptide was called SHe-CC4S. After purification of the mHBc-SHe-VLPs, by size exclusion chromatography, the degree of crosslinking was examined by SDS PAGE. Figure 7 illustrates that approximately 50% of the HBc proteins is chemically linked to a SHe-CC4S peptide. The slower migrating bands likely represent mHBc monomers to which 2 or 3 SHe(cc4s) peptides were linked. To test if SHeCC4S linked mHBc proteins still assemble into VLPs of the expected size (30-34 nm) Dynamic Light Scattering analyses was performed on the generated mHBc-SHe partikels and the 1604 M2e-HBc VLP as fully functional reference. Figure 8 illustrates that the size distribution of mHBc-SHe-CC4S overlaps
with that of the 1604 M2e-HBc control, with a maximum at 30 nm, which corresponds with the reported size of HBc VLPs (Clarke et al., 1987).

### Example 4: Design, construction and purification of SHe-tGCN4-Flag

Next to presenting the SHe peptide at the surface of mHBc VLP's, SHe was also fused to tGCN4, which is known to induce a strong humoral response towards fused peptides (Ref marina GCN4). SHe and a Flag-tag were genetically linked to respectively the 5'end and the 3'end of the tGCN4 coding sequence and cloned into a PLT32 expression vector. After expression in E. coli, recombinant SHe-tGCN4-Flag was purified by anion exchange, hydrophobic interaction and gel filtration chromatography (Figure 9).

### Example 5: mHBc-SHe(CC4S) and SHe-tGCN4 vaccination induces SHe specific antibodies and protection against RSV infections

To test if vaccination with mHBc-SHe(CC4S) and SHe-tGCN4 can evoke protection against RSV infections, Balb/c mice were vaccinated three times intranasal with 10 ug mHBc-SHe(CC4S) and SHe-tGCN4 in combination with 1ug LTR192G adjuvant. PBS and empty mHBc, the later in combination with 1ug LTR192G, were used as negative controls. Immunizations were performed every 3 weeks. A single RSV infection (5. 10⁵ PFU) was used as positive control. Between the second and the third week after each immunization, blood was collected to investigate the induction of SHe specific IgG antibodies. The presence of She specific antibodies was tested by SHe peptide ELISA. Figure 10A demonstrates that SHe peptide specific IgG antibodies are induced and boosted after respectively the second and third immunization with mHBc-SHe(CC4S) and SHe-tGCN4. Three successive Flag-COMPcc- SHe/LTR192G immunizations resulted in high levels of IgG2a SHe-specific antibodies and somewhat lower levels of IgG1 SHe-specific antibodies, indicating a Th1 oriented/driven immune response (Figure 10 B).

To test if vaccination with mHBc-SHe(CC4S) or SHe-tGCN4 can hamper RSV infection, the mice were challenged with 5.10⁶ PFU RSV-A2 3 weeks after the last boost immunization. Three days after challenge the mice were sacrificed to determine the pulmonary RSV-A2 levels by QPCR. Figure 11 shows that all mice that were vaccinated with mHBc-SHe(CC4S) or SHe-tGCN4 or mice that were infected beforehand with RSV have lower pulmonary levels of genomic RSV RNA than mice that were vaccinated with mHBc. These data confirm our previous observation that mucosal SHe based vaccination can partially protect mice against RSV replication. Remarkably, all mice that were vaccinated with an empty mHBc in combination with the LTR192G adjuvant, displayed lower levels of RSV than mice that were immunized with PBS without LTR192G adjuvant. This might be explained by the effect of
LTR192G on the mouse innate immune system. The E. coli Heat labile entertoxin has been shown to provide generic protection against lung viral infections, including RSV via innate imprinting (ref Williams and Hussel 2004). The effect of innate imprinting by LTR192G on lung viral replication appears to be transient as the impact of TLR192R on RSV replication is strongly reduced when viral infection occurs nine weeks after the last LTR192G administration. Again none of the mice showed significant bodyweight loss, indicating that vaccination with SHe when presented by VLP's or tGCN4 is not inducing enhancement of disease upon challenge (Figure 12).

### Example 6: Production and testing of SHe specific monoclonal antibodies.

To investigate if SHe specific antibodies that can interact with infected cells, can provide protection against RSV infections, we developed RSV SHe specific monoclonal antibodies based on SHe-TGCN4 immunized mice. One IgG1 (3D11) and one IgG2a (3G8) subtype hybridoma that produced antibodies that efficiently bound to SHe peptide in an ELISA were selected, subcloned and used for antibody production. The 3D11 and 3G8 were purified via protein A affinity chromatography and tested for binding efficacy to SHe via an ELISA. Figure 13 shows that 3D11 and 3G8 can bind to coated SHe peptide and are respectively of the IgG1 and IgG2a subtype.

As antibodies can protect against viral infections via recognition and killing of infected cells by (ADCC) or CDC, we investigated if the SHe specific mAbs 3D11 and 3G8 can recognize SH at the surface of cells. Therefore, Hek293T cells were transfected with an RSV SH expression vector or with a control Firefly luciferase vector (Schepens et al., 2005), both in combination with a GFP expression vector. Twenty four hours after transfection, live cells were stained with different concentrations of the SHe specific monoclonal antibodies (3D11 and 3G8) or isotype matched Influenza M2e specific antibodies (14C2 IgG1 and a IG2a M2e specific mAb). Polyclonal serum form Flag-COMPcc-SHe immunized mice was used as positive control. Figure 14 demonstrates that Flag-COMPcc-SHe polyclonal serum but also both 3D11 and 3G8 mAbs can readily bind to SH expressing cells but not to control cells. In contrast the neither the IgG1 and IgG2a Influenza M2e specific antibodies could bind to SH expressing cells. These data clearly demonstrate that both 3D11 and 3G8 can recognize the ectodomain of SH expressed at the surface of cells.

During infection the RSV SH protein is mainly expressed at the ER, golgi and cell membrane. So to more directly investigate if the RSV SH specific antibodies can recognize infected cells via SH, expressed at the surface of these cells we performed immunostaining of RSV infected and mock infected cells. Human A594 lung epithelial cells were either infected with 0.05 MOI of RSV or mock infected. Twenty four hours after infection the cells were fixed and stained with the SHe specific mAbs 3D11 or 3G8 in combination with polyclonal anti-RSV immune serum.

Figure 15 illustrates that the SHe specific mABs 3D11 and 3G8 can readily recognize SH at the cell membrane and near the nucleus (likely corresponding to ER and Golgi) of infected cells. This indicates that SHe mAbs protect against RSV infection by recognizing RSV infected cells. In this way, the here described SHe mAbs 3D11 and 3G8 can be used as prophylactic or therapeutic treatment.

### Example 7: passive immunization using SHe specific mAB 3G8 reduces RSV replication.

To test if SHe specific antibodies can reduce RSV replication in vivo, mice were passively immunized with SHe specific monoclonal antibodies. SHe specific 3G8 monoclonal antibodies, isotype control antibodies or PBS were intranasal administered to mice one day before and one day after RSV Challenge. Three days after RSV challenge blood was collected to test for the presence of mAb's in the serum of the treated mice. Four days after RSV challenge the mice were sacrificed to determine the viral titer in the lungs. Peptide ELISA demonstrated the presence of low concentrations of SHe specific and isotype control antibodies in the serum of mice treated with the respective antibodies (data not shown). Figure 16 illustrates that mice that received SHe specific monoclonal antibodies have reduced lung RSV titers as compared with mice that were treated with PBS or isotype control monoclonal antibodies. These data suggest that intranasal administration of SHe specific antibodies can reduce RSV infection in mice.

### Example 8: construction of SHe-KLH

To test if SHe-based vaccines can also protect against RSV infections when this vaccine is administered via an alternative route with an alternative adjuvant and with a different carrier, the vaccine was tested intraperitoneally, with keyhole limpet hemocyanin (KLH) as a carrier. Maleimide-activated KLH (Pierce) was chemically linked to the peptide (CGGGSNKLSEYNVFHNKTFELPRARVNT (SEQ ID N° 50); the sequence corresponding to the RSV A SH ectodomain (She) is underlined) corresponding to the RSV A SH ectodomain. To promote directional chemical linking a CysGlyGlyGlySer linker was added to the N-terminus of the RSV A SHe peptide. In addition the cysteine residue present in the natural RSV A SHe was substituted by a Serine residue. Chemical linkage was performed according to the manufacturers' instructions (Pierce). Cross linked KLH-SHe proteins were isolated by size exclusion chromatography.

### Example 9: Intraperitoneal vaccination with KLH-SHe reduces RSV replication in mice.

To test if intraperitoneal (I.P.) vaccination with a SHe-based vaccine can evoke protection against RSV infections, Balb/c mice (six mice per group) were vaccinated three times intraperitoneally with 20 µg of KLH-SHe or KLH, each in combination with 50 µl of Incomplete Freunds' Adjuvant (Milipore). PBS vaccination without adjuvant was used as an additional negative control. Between the second and third week after vaccination, blood was collected to determine the induction of SHe-specific IgG antibodies. The presence of SHe-specific antibodies was determined and quantified by SHe peptide ELISA. Figure 17 (A-B) demonstrates that 3 successive vaccinations with KLH-SHe induces high levels of SHe-specific IgG antibodies of both the IgG1 and IgG2a subtype. No SHe specific IgG antibodies could be detected in sera form PBS or KLH vaccinated mice. In addition flow cytometric analysis revealed that serum derived from mice that had been vaccinated intraperitoneally with KLH-SHe can specifically bind to HEK293T cells that express the RSV SH protein at their surface, whereas pre-immune serum did not.

To test whether intraperitoneal KLH-SHe vaccination can reduce RSV infection, the vaccinated mice were infected with 1.10⁶ PFU of RSV-A2, 4 weeks after the last vaccination. Five days after challenge, the mice were sacrificed to determine the pulmonary RSV-A2 titer by plaque assay. Figure 17D illustrates that significantly less virus could be detected in the lungs of SHe- KLH vaccinated than in the lungs of KLH vaccinated mice (P > 0,005, Mann-Whitney *U* test). The observation that among KLH-SHe vaccinated mice, higher titers of serum SHe-specific IgG antibodies strongly correlated (R² = 0,95) with lower levels of pulmonary RSV at day 5 post infection, suggests that reduction of RSV replication by KLH-SHe vaccination is mediated by SHe specific antibodies (Figure 17E). The body weight of all mice was monitored at the day of infection and the day of sacrifice. Figure 17C illustrates that mice that were vaccinated with KLH-SHe gained significantly more weight than mice that were vaccinated with KLH (P > 0,005, Mann-Whitney *U* test). These data demonstrate that intraperitoneal vaccination with a SHe-based vaccine can reduce RSV replication without inducing morbidity. In addition these data illustrate that next to mHBc, tGCN4 and COMPcc also KLH can be used as a protein carrier for SHe peptide based vaccines. Moreover these data illustrate that next to Titermax, also Incomplete Freunds' Adjuvant can be used as an appropriated adjuvant to induce SHe- specific immunity.

### Example 10: Intranasal vaccination with KLH-SHe reduces RSV replication in mice.

To test if intranasal vaccination with KLH-SHe can evoke protection against RSV infections, Balb/c mice (six mice per group) were vaccinated three times intranasally with 20 µg of KLH- SHe or KLH, each in combination with 1 µg of LTR192G adjuvant. PBS vaccination without adjuvant was used as an additional negative control. Between the second and third week after vaccination a, blood was collected to investigate the induction of SHe-specific IgG antibodies.

The presence of SHe- specific antibodies was tested by SHe peptide ELISA. Figure 18 (A-B) demonstrates that 3 successive vaccination with KLH-SHe induces SHe-specific IgG antibodies of both the IgG1 and IgG2a subtype. No SHe-specific IgG antibodies could be detected in sera form PBS or KLH vaccinated mice. In addition flow cytometric analysis revealed that serum derived from mice that were vaccinated intranasally with KLH-SHe serum but not pre-immune serum can specifically bind to HEK293T cells that express the RSV SH protein at their surface.

To test whether intraperitoneal KLH-SHe vaccination can reduce RSV infection, the vaccinated mice were infected with 1.10⁶ PFU of RSV-A2, 9 weeks after the last vaccination. Five days after challenge, the mice were sacrificed to collect BAL (Broncho Alveolar Lavage) fluid (3ml). The RSV-A2 titer in the collected BAL fluids was determined by plaque assay. Figure 18E illustrates that significantly less virus could be detected in the lungs of KLH-SHe vaccinated than in the lungs of KLH vaccinated mice (P > 0,05, Mann-Whitney *U* test). The presence of SHe-specific IgA and IgG antibodies in the collected BAL fluids was analyzed by SHe peptide ELISA. This analysis revealed that in contrast to PBS and KLH vaccinated mice, the BAL fluids of mice vaccinated with KLH-SHe contained both IgG and IgA SHe-specific antibodies (Figure 18C-D). The levels of IgG SHe-specific antibodies present in the BAL fluid of KLH-SHe vaccinated mice correlated with the levels of IgG SHe- specific antibodies in the serum of the respective mice. The observation that among KLH-SHe vaccinated mice, higher titers of SHe- specific IgG antibodies present in the BAL fluid strongly correlate (R² = 0,97) with lower levels of pulmonary RSV titers on day 5 post infection, suggests that reduction of RSV replication by KLH-SHe vaccination is mediated by SHe-specific antibodies (Figure 18 F). These data demonstrate that intranasal vaccination with a SHe-based vaccine can reduce RSV replication without inducing morbidity. In addition these data confirm that next to mHBc, tGCN4 and COM Pec also KLH can be used as a protein carrier for SHe peptide based vaccines.

### Example 11: Passive transfer of KLH-SHe immune serum protects against RSV infection in mice.

To further investigate if the reduction in RSV replication, in mice that have been vaccinated with a SHe-based vaccine, can be mediated by RSV SHe-specific antibodies, a passive transfer experiments were performed. Balb/c mice were vaccinated intraperitoneally with 20µg of either KLH-SHe or KLH, both in combination with 75 µl of Incomplete Freund adjuvant. As an additional negative control, mice were vaccinated with PBS without adjuvant. SHe peptide ELISA illustrated that the sera of all mice that had been vaccinated with KLH-SHe contains high levels of SHe-specific IgG antibodies. After final bleeding the sera of the mice of each
group were pooled and heat inactivated at 56°C for 30 minutes. To test if KLH-SHe sera can protect against RSV infections, 40 µl of KLH or KLH-SHe sera were administered to mice intranasally one day before (day -1) and one day after (day 1) RSV challenge (2.10⁵ PFU) (day 0). Mice that were treated with PBS were included as additional controls. The weight of all mice was monitored daily (Figure 19C). Five days post infection the mice were sacrificed to prepare lung homogenates. Plaque assay analysis demonstrated that the lung homogenates of mice that had been treated with KLH-SHe serum contained about 40 times less (ratio of means of viral titers) replicating virus than the lung homogenates originating form mice treated with KLH serum (Figure 19 B). The observation that the pulmonary RSV titer of mice that were treated with KLH serum did not differ from the pulmonary RSV titer of mice that were treated with PBS, illustrates that administration of control serum does not impact pulmonary RSV replication in mice.

### Example 12: construction of mHBc-SHeB

Although highly conserved within their subtype, the SHe amino acid sequences of RSV B viruses differs from that of the RSV A subtype viruses. Therefore, to protect against RSV B viruses a SHe-based vaccine most likely needs to include the RSV B SHe amino acid sequence.

A RSV B SHe vaccine was constructed by chemicaly linking the consensus RSV B SHe peptide (SHeB: CGGGSNKLSEHKTFSNKTLEQGQMYQINT (SEQ ID N° 51) to the mHBc virus like particles. To promote chemical linking a CysGlyGlyGlySer linker was added to the N- terminus of the RSV B SHe peptide. In addition the cysteine residue present in the natural RSV B SHe was substituted by a serine residue. The immunogen resulting from chemical linkage of the RSV B She peptide to mHBc was named mHBc-SHeB. After purification of the mHBc-SHeB VLPs by size exclusion chromatography, the degree of crosslinking was analyzed by SDS-PAGE gel elektrophoresis and commassie staining. Figure 20 illustrates that more than half of the HBc monomers are crosslinked to at least one SHe peptide.

### Example 13: immunization of mice with mHBc-SHeB induces SHeB specific Abs that bind to the surface of RSV B infected cells.

To test whether mHBc-SHeB VLPs were immunogenic one BALB/c mouse was immunized three times subcutaneously with 20µg of mHBc-SHeB combined with 50 µl Titermax (Sigma). The thee immunizations were performed with two weeks intervals. Bleedings were performed one day before each immunization and two weeks after the final immunization. To test whether mHBc-SHeB immune serum can recognize RSV B SH proteins expressed on the surface of
infected cells, Vero cells were either mock infected or infected with a clinical isolate of RSV B virus (kindly provided by Dr. Marc van Ranst, University of Leuven, Leuven, Belgium). Seventy-two hours after infection the cells were fixed and either permeabilized using 0,2 % Triton X-100 or not permeabilized. The cells were then stained with either mHBc-SHeB immune serum (1/100 dilution) or control immune serum (1/100 dilution) derived from BALB/c mice that had been vaccinated with KLH (KLH serum) in combination with incomplete Freund adjuvant. The samples were analyzed by immunofluoresence microscopy or flow cytometry. Figure 21A and B illustrates that mHBc-SHeB immune serum can bind to both permeabilized and non-permeabilized RSV B infected cells but not to non-infected cells. In contrast control immune serum did not bind to RSV B infected cells. This demonstrates that vaccination of mice with mHBc-SHeB induces serum antibodies that can recognize RSV B infected cells, most likely by binding to the RSV B SH protein that is expressed at the surface of RSV B infected cells.

### Example 14. mHBc-SHeB immunization reduces RSV replication in mice.

To test whether mHBc-SHeB vaccination can protect mice from RSV B infection, two groups of 6 mice were immunized with mHBc or mHBc-SHeB VLPs, adjuvanted with 50 µl of incomplete Freund adjuvant. As additional controls six mice were vaccinated with PBS. Vaccinations were performed intraperitoneally, three times with three weeks interval. Bleedings were performed two weeks after each immunization. The induction of SHe-specific antibodies was determined by peptide ELISA using SHeA or SHeB as coating peptides. This analysis demonstrated that in all mice three successive mHBc-SHeB immunizations induced high titers of RSV B She- specific IgG antibodies of both IgG1 and IgG2a subtype (Figure 22A-C). mHBc-SHeB immune serum also bound to the SHeA peptide but to a much lower extend (Figure 22A, B and D).

Previous experiments in our and other laboratories have illustrated that no or very little replicating virus can be rescued from RSV B infected mice. Nevertheless, we could observe that infections with clinical RSV B isolates induce pulmonary inflammation and weight loss in BALB/c mice (data not shown). Therefore, we tested whether mHBc-SHeB vaccination could protect mice from RSV B-induced pulmonary inflammation. Six days after intranasal challenge of mice with 2.10⁶ PFU of an RSV B clinical isolate Broncho Alveolar Lavage (BAL) was performed. Mock infected mice were used as negative control for analysis of BAL cell infiltration. The BAL fluid was analyzed for immune cell infiltration by flow cytometry as described in Bogaert et al., 201 1. Figure 22E-F shows that RSV B infection results in pulmonary infiltration of immune cells, especially CD8⁺ T lymphocytes which are known to be responsible for RSV induced morbidity in mice. However, compared to PBS or mHBc
vaccinated mice, mHBc-SHeB vaccinated mice displayed significantly lower pulmonary cell infiltration. These data demonstrate that mHBc-SHeB vaccination reduces RSV related immune pathology.

### Example 15: design, expression and purification of the LPP₍₅₎-SHe protein.

As an alternative protein scaffold to present SHe as a pentamer we used the pentameric tryptophan-zipper described by Liu et.al. (LPP₍₅₎), which is derived from the *E. coli* LPP-56 lipoprotein (Liu et al., 2004). The coding sequence of the LPP₍₅₎ tryptophan-zipper was genetically fused to the SHe coding sequence and cloned into an *E. coli* expresson vector (pLH36) containing a hexahistidine peptide and a caspase cleavage site as described by Mertens et al., 1995. This expression plasmid was named pLH36-HisDEVD-LPP-SHe (SEQ ID N° 49). Expression from this plasmid renders the chimeric LPP₍₅₎-SHe protein (SEQ ID N° 52) (MHHHHHHPGGS*DEVD***AKWDQWSSDWQTWNAKWDQWSNDWNAWRSDWQ AKDDWARWNQRWDNWAT***GG**NKLCEYNVFHNKTFELPRARVNT***, His-tag sequence is underlined, linkers are in italic, DEVD caspase cleavage site is in italic + underlined, pentameric LPP tryptophan-zipper is in bold and the RSV A SH ectodomain is in bold + italic). After induction of expression in E. coli the LPP₍₅₎-SHe protein was purified by subsequent Nickel affinity, anion- exchange and gel filtration chromatography. Figure 23 demonstrates that the LPP₍₅₎-SHe protein, can be recognized by She-specific 3G8 monoclonal antibodies, both in a crude cell extract (23A) and as a purified protein (24B).

### Example 16: Cotton rat immunization

In order to prove the efficacy of the vaccine in an independent animal model, cotton rats are used. Cotton rats (*Sigmondon hispidus*) are susceptible to RSV infection (Prince et al., 1978). Five groups of six cotton rats each are used. Two group of animals are immunized intraperitoneally (i.p.) with 100 µg of KLH (vehicle control) or 100 µg of KLH-SHe (i.e. a chemical conjugate of SHe peptide derived from RSV-A with KLH as a carrier). KLH and KLH- SHe vaccine antigens are formulated with incomplete Freund's adjuvant and used to immunize cotton rats on days 0, 21, and 42. A third group of animals is immunized intramuscularly with formalin-inactivated RSV (FI-RSV) in the presence of alum adjuvant. The latter group serves as a positive control for the induction of vaccine-enhanced disease that becomes apparent upon subsequent challenge with RSV. A fourth group is infected with 2.04 x 10⁵ plaque forming units per cotton rat of RSV-Tracy on day 0 and serves as positive control for protection against subsequent challenge. A fifth group of cotton rats remains untreated until the day of challenge
and served as control for the challenge with RSV. The schedule of the vaccination is shown in figure 24.

Sera are collected before each immunization and on the day of challenge. On day 63, cotton rats are challenged intranasally with 2.04 x 10⁵ plaque forming units of RSV-Tracy. The challenge virus is administered intranasally in a volume of 100 microliter while the animals are lightly anesthetized with isoflurane. On day 68, serum is collected and all animals are sacrificed to collect lungs for virus titration and histopathological analysis. Each lung is divided in two to perform histopathological analysis and virus titration. The left lungs are tied off an used for histopathological analysis. The lobes of the right lung are lavaged using 3 ml of Iscove's media with 15% glycerin. The lavage fluid is stored on ice until titration. In addition, nasal lavages are prepared with 2 ml (1 ml for each nare) in the same medium.

The viral load in the lung and nasal lavages is determined by plaque assay on HEp2 cells. Cells are infected for 90 min with a serial dilution of the lavage samples. After removal of the inoculum the cells are overlayed with 2% methylcellulose in MEM containing antibiotics. After 6 days of incubation at 37°C in a C0₂-incubator, plaques are counterstained with 0.1 % crystal violet/10% formalin solution and left at room temperature for 24 hours.

For histopathological analysis, the left lung is perfused with 10% neutral buffered formalin. Fixed lung tissue is subsequently processed with a microtome to produce sections that are stained with hematoxilin and eosin and scored for the degree of histopathological lesions. Serum samples are assayed for the presence of anti-SHe and anti-RSV neutralizing antibodies by peptide ELISA and by a microneutralization assay. For peptide ELISA, plates are coated overnight at 37°C with 2 µg of SHe-peptide in 50 µl of 0,1 M carbonate buffer pH 9,6. After coating, plates are blocked with 3% (w/v) milk powder in PBS, followed by application of 3-fold serial dilutions on cotton rat sera. Retained SHe-specific cotton rat IgG are detected using horseradish peroxidase conjugated secondary antibodies and tetramethylbenzidine substrate. The endpoint anti-SHe peptide IgG titer in the samples is defined as the highest dilution for which the absorbance is at least twice as high as that of the preimmune serum.

Neutralizing antibody titers are determined for RSV-A and -B in 96-well microtiter plates with HEp2 cells. Serial dilutions of serum samples are mixed with a fixed amount of inoculum virus. The neutralizing antibody titer s defined as the serum dilution at which > 50% reduction is cytopathic effect is observed. This cytopathic effect refers to the destruction of cells and is determined visually after the cells are fixed with 10% neutral buffered formalin and stained with crystal violet. The results show that the animals, vaccinated with KLH-SHe in Freunds adjuvant develop neutralizing antibodies and are clearly protected, whereas the vehicle control shows no protection at all.

### REFERENCES

- Altschul, S.F., Madden, T.L., Schaffer, A.A., Zhang, J., Zhang, Z., Miller, W. and Lipman, D.J. (1997). Gapped BLAST and PSI-BLAST: a new generation of protein database search programs, Nucleic Acids Res. 25:3389-3402.
- Beeler, J.A., van Wyke Coelingh, K. (1989). Neutralization epitopes of the F glycoprotein of respiratory syncytial virus: effect of mutation upon fusion function. J. Virol. 63, 2941-2950.
- Bocchini, J. A. Jr, Bernstein, H.H., Bradley, J.S., Brady, M.T., Byington, C.L., Fisher, M.C., Glode, M.P., Jackson, M.A., Keyserling, H.L., Kimberlin, D.W., Orenstein, W.A., Schutze, G.E., Willoughby, R.E., Dennehy, P.H., Frenck, R.W. Jr, Bell, B., Bortolussi, R., Clover, R.D., Fischer, M.A., Gellin, B., Gorman, R.L., Pratt, R.D., Lee, L, Read, J.S., Starke, J.R., Swanson, J., Baker, C.J., Long, S.S., Pickering, L.K., Ledbetter, E.O., Meissner, H.C., Rubin, L.G., Frantz, J. (2009). From the American Academy of Pediatrics: Policy statements-Modified recommendations for use of palivizumab for prevention of respiratory syncytial virus infections. Pediatrics, 124, 1694-16701.
- Boisgerault, F., Moron, G. and Leclerc, C. (2002). Virus-like particles: a new family of delivery systems. Expert Rev Vaccines 1, 101-109.
- Bogaert, P., Naessens, T., De Koker, S., Hennuy, B., Hacha, J., Smet, M., Cataldo, D., Di Valentin, E., Piette, J., Tournoy, K.G. and Grooten, J. (2011). Inflammatory signatures for eosinophillic vs. neutrophillic allergic pulmonary inflammation reveal critical regulatory checkpoints. Am. J. Physiol. Lung Cell Mol. Physiol., 300, L679-690.
- Clarke, B.E., Newton, S.E., Carroll, A.R., Francis, M.J., Appleyard, G., Syred, A.D., et al. (1987). Improved immunogenicity of a peptide epitope after fusion to hepatitis B core protein. Nature 330, 381-384.
- De Filette, M., Min Jou, W., Birkett, A., Lyons, K., Schultz, B., Tonkyro, A., et al. (2005). Universal influenza A vaccine: optimization of M2-based constructs. Virology 337, 149-161.
- De Filette, M., Martens, W., Roose, K., Deroo, T., Vervalle, F., Bentahir, M., Vandekerckhove, J., Fiers, W. and Saelens, X. (2008). An influenza A vaccine based on tetrameric ectodomain of matrix protein 2. J. Biol. Chem., 283, 11382-11387.
- Falsey, A.R., Cunningham, C.K., Barker, W.H., Kouides, R.W., Yue.n J.B., Menegus, M., Weiner, L.B., Bonville, C.A., and Betts, R.F. (1995). Respiratory syncytial virus and influenza A infection in the hospitalized elderly. J. Infect. Dis. 172, 389-394.
- Falsey, A.R. and Walsh, E.E. (1996). Safety and immunogenicity of a respiratory syncytial virus subunit vaccine (PFP-2) in ambulatory adults over age 60. Vaccine 14, 1214-1218.
- Falsey, A.R. and Walsh, E.E. (1997). Safety and immunogenicity of a respiratory syncytial virus subunit vaccine (PFP-2) in the institutionalized elderly. Vaccine, 15, 1130-1132.
- Gimenez, H.B., Keir, H.M. and Cash, P. (1987) Immunoblot analysis of the human antibody response to respiratory syncytial virus infection. J. Gen. Virol. 68, 1267-1275.
- Groothuis, J.R., King, S.J., Hogerman, D.A., Paradiso, P.R. and Simoes, E.A. (1998). Safety and immunogenicity of a purified F protein respiratory syncytial virus (PFP-2) vaccine in seropositive children with bronchopulmonary dysplasia. J. Infect. Dis. 177, 467-469.
- Hacking D. and Hull, J. (2002). Respiratory Syncytical virus - viral biology and the host response. J. Infection 45, 18-24.
- Hermanson, G.T. (1996). Bioconjugate techniques. Academic Press, Inc. 525 B street, San Diego, CA and Academic Press Limited, Oval Road, London, UK. ISBN-0-12- 342335-X.
- Jegerlehner, A., A. Tissot, F. Lechner, P. Sebbel, I. Erdmann, T. Kundig, T. Bachi, T. Storni, G. Jennings, P. Pumpens, W.A. Renner, and M.F. Bachmann. (2002). A molecular assembly system that renders antigens of choice highly repetitive for induction of protective B cell responses. Vaccine. 20:3104-12.
- Kapikian, A.Z., Mitchell, R.H., Chanock, R.M., Shvedoff, R.A. and Stewart, C.E. (1969). An epidemiologic study of altered clinical reactivity to respiratory syncytial (RS) virus infection in children previously vaccinated with an inactivated RS virus vaccine. Am. J. Epidemiol. 89, 405-421.
- Karron, R.A., Wright, P.F., Belshe, R.B., Thumar, B., Casey, R., Newman, F., Polack, F.P., Randolph, V.B., Deatly, A., Hackell, J., Gruber, W., Murphy, B.R. and Collins, P.L. (2005). Identification of a recombinant live attenuated respiratory syncytial virus vaccine candidate that is highly attenuated in infants. J. Inf. Dis. 191, 1093-1104.
- Liu, J., W. Yong, Y. Deng, N.R. Kallenbach, and M. Lu. (2004). Atomic structure of a tryptophan-zipper pentamer. Proc Natl Acad Sci U S A. 101:16156-61.
- Liu, J., Q. Zheng, Y. Deng, N.R. Kallenbach, and M. Lu. (2006). Conformational transition between four and five-stranded phenylalanine zippers determined by a local packing interaction. J Mol Biol. 361 :168-79.
- Malashkevich, V.N., Kammerer, R.A., Efimov, V.P., Schulthess, T. and Engel, J. (1996). The crystal structure of a five-stranded coiled coil in COMP: a prototype ion channel? Science 274, 761-765.
- McFarlane, A. A., Orriss, G.L. and Stetefeld, J. (2009). The use of coiled-coil proteins in drug delivery systems. Eur. J. Pharmacol. 625, 101-107.
- Mertens, N., Remaut, E. and Fiers W. (1995). Versatile, multi-featured plasmids for high-level expression of heterologous genes in Escherichia coli: overproduction of human and murine cytokines. Gene, 164, 9-15.
- Meyer, G., Deplanche, M. and Schelcher, F. (2008). Human and bovine respiratory syncytial virus vaccine research and development. Comp Immunol Microbiol Infect Dis. 31 , 191-225.
- Murata, Y. (2009) Respiratory Syncytial Virus vaccine development. Clin. Lab. Med. 29, 725-739.
- Neirynck, S., T. Deroo, X. Saelens, P. Vanlandschoot, W.M. Jou, and W. Fiers. (1999). A universal influenza A vaccine based on the extracellular domain of the M2 protein. Nat Med. 5: 1157-63.
- Norton, E.B., J.D. Clements, T.G. Voss, and L. Cardenas-Freytag. (2010) Prophylactic administration of bacterially derived immunomodulators improves the outcome of influenza virus infection in a murine model. J Virol. 84:2983-95.
- Orga, P.L. (2004). Respiratory syncytial virus: the virus, the disease and the immune response. Pediatric respiratory reviews, 5, suppl.A, S119-S126.
- Olmsted, R.A., and P.L. Collins. (1989). The 1A protein of respiratory syncytial virus is an integral membrane protein present as multiple, structurally distinct species. J Virol. 63:2019-29
- Power, U.F., Nguyen, T.N., Rietveld, E., de Swart, R.L., Groen J., Osterhaus, A.D., de Groot, R., Corvaia, N., Beck, A., Bouveret-le-Cam, N and Bonnefoy, J.Y. (2001). Safety and immunogenicity of a novel recombinant subunit Respiratory Syncytial Virus vaccine (BBG2Na) in healthy young adults. J. Infect. Dis. 184, 1456-1460.
- Prescott, W.A. Jr., Doloresco, F., Brown. J. and Paladino, J.A. (2010). Cost effectiveness of respiratory syncytial virus prophylaxis: a critical and systematic review. Pharmacoeconomics, 28, 279-293.
- Prince GA, Jenson AB, Horswood RL, Camargo E, Chanock RM., The pathogenesis of respiratory syncytial virus infection in cotton rats, American Journal of Pathology, 1978, 93, 771-791.
- Prince, G.A., Jenson, A.B., Hemming, V.G., Murphy, B.R., Walsh, E.E., Horswood, R.L., et al. (1986) Enhancement of respiratory syncytial virus pulmonary pathology in cotton rats by prior intramuscular inoculation of formalin-inactiva ted virus. J Virol 57, 721-728.
- Schepens, B., S.A. Tinton, Y. Bruynooghe, R. Beyaert, and S. Cornells. (2005). The polypyrimidine tract-binding protein stimulates HIF-1 alpha IRES-mediated translation during hypoxia. Nucleic Acids Res. 33:6884-94.
- Schmidt, A.C., Wenzke, D.R., McAuliffe, J.M., St Claire, M., Elkins, W.R., Murphy, B.R. and Collins, P.L. (2002). Mucosal immunization of rhesus monkeys against respiratory syncytial subgroups A and B and human parainfluenza virus type 3 by living cDNA- derived vaccine based on a host-range attenuated bovine parainfluenza virus type 3 vector backbone. J. Virol. 76, 1089-1099.
- Shu, W., Liu, J., Ji, H. and Lu, M. (2000). Core structure of the outer membrane lipoprotein from Escherichia coli at 1.9 A resolution. J Mol Biol. 299, 1101-11 12.
- Slütter, B., Soema, P.C., Ding, Z., Verheul, R., Hennink, W. And Jiskoot, W. (2010). Conjugation of ovalbumin to trimethul chitosan improves immunogenicity of the antigen. J. Controlled Release, 143, 207-214.
- Timmerman, P, Puijk, W.C. and Meloen, R.H. (2007). Functional reconstruction and synthetic mimicry of a conformational epitope using CLIPS™ technology. L. Mol. Recognition 20, 283-299.
- Tsutsumi, H., Honjo, T., Nagai, K., Chiba, Y., Chiba, S., and Tsuguwa, S. (1989). Immunoglobulin A antibody response to respiratory syncytial virus structural proteins in colostrums and milk. J. Clinical Microbiol. 27, 1949-1951.
- Whitacre, D.C., Lee, B.O. and Milich, D.R. (2009). Use of hepadnavirus core proteins as vaccine platforms. Expert Rev Vaccines. 8, 1565-1573. Schepens, B., S.A. Tinton, Y. Bruynooghe, R. Beyaert, and S. Cornells. 2005. The translation during hypoxia. Nucleic Acids Res. 33:6884-94.
- Williams, J. P., D.C. Smith, B.N. Green, B.D. Marsden, K.R. Jennings, L.M. Roberts, and J.H. Scrivens. (2006). Gas phase characterization of the noncovalent quaternary structure of cholera toxin and the cholera toxin B subunit pentamer. Biophys J. 90:3246-54.

## Claims

1. A vaccine for use in a method of vaccination against RSV infection, the vaccine comprising an immunogenic composition and an adjuvant, **characterized in that** the immunogenic composition consists of the ectodomain of the small hydrophobic protein of a Respiratory Syncytial Virus (RSV) and a carrier, wherein the carrier is a molecule that is heterologous to the small hydrophobic protein of the Respiratory Syncytial Virus (RSV) and further wherein said ectodomain is chemically or genetically linked to the carrier.

2. The vaccine for use according to claim 1, wherein said ectodomain is presented as an oligomer.

3. The vaccine for use according to claim 1 or 2, wherein said ectodomain consists of SEQ ID N° 1 or SEQ ID N° 2.

4. The vaccine for use according to claim 2, wherein said oligomer is a pentamer.

5. The vaccine for use according to claim 1, wherein said carrier is selected from the group consisting of Cartilage Oligomeric Matrix Protein (comp), Lpp-56, and a virus-like particle.

6. The vaccine for use according to claim 1, wherein said carrier is a non-proteinaceous carrier.

7. The vaccine for use according to claim 6, wherein said non-proteinaceous carrier is a liposome or a trimethyl chitosan.

8. An antibody specific to the ectodomain of the small hydrophobic protein of RSV, for use in a method of preventing or treating RSV infection.

9. A pharmaceutical composition, comprising a monoclonal antibody according to claim 8, for use in a method of preventing or treating RSV infection.

## Patentansprüche

1. Impfstoff zur Verwendung in einem Verfahren zur Impfung gegen eine RSV-Infektion, wobei der Impfstoff eine immunogetische Zusammensetzung und ein Adjuvans umfasst, **dadurch gekennzeichnet, dass** die immunogetische Zusammensetzung aus der Ektodomäne des kleinen hydrophoben Proteins eines respiratorischen Synzytial-Virus (RSV) und einem Träger besteht, wobei der Träger ein Molekül ist, welches heterolog zu dem kleinen hydrophoben Protein des respiratorischen Synzytial-Virus (RSV) ist, und wobei des Weiteren die Ektodomäne chemisch oder genetisch mit dem Träger verbunden ist.

2. Impfstoff zur Verwendung nach Anspruch 1, wobei die Ektodomäne als Oligomer vorliegt.

3. Impfstoff zur Verwendung nach Anspruch 1 oder 2, wobei die Ektodomäne aus SEQ ID N°1 oder SEQ ID N°2 besteht.

4. Impfstoff zur Verwendung nach Anspruch 2, wobei das Oligomer ein Pentamer ist.

5. Impfstoff zur Verwendung nach Anspruch 1, wobei der Träger aus der Gruppe bestehend aus dem oligomeren Matrix-Protein des Knorpels (Cartilage Oligomeric Matrix Protein, COMP), Lpp-56 und einem virusähnlichen Partikel ausgewählt ist.

6. Impfstoff zur Verwendung nach Anspruch 1, wobei der Träger ein nichtproteinischer Träger ist.

7. Impfstoff zur Verwendung nach Anspruch 6, wobei der nicht-proteinische Träger ein Liposom oder ein Trimethylchitosan ist.

8. Antikörper, der für die Ektodomäne des kleinen hydrophoben Proteins von RSV spezifisch ist, zur Verwendung in einem Verfahren zum Verhindern oder zur Behandlung einer RSV-Infektion.

9. Pharmazeutische Zusammensetzung mit einem monoklonalen Antikörper nach Anspruch 8, zur Verwendung in einem Verfahren zum Verhindern oder zur Behandlung einer RSV-Infektion.

## Revendications

1. Vaccin destiné à être utilisé dans un procédé de vaccination contre une infection par le VRS, le vaccin comprenant une composition immunogène et un adjuvant, **caractérisé en ce que** la composition immunogène est constituée de l'ectodomaine de la petite protéine hydrophobe d'un Virus Respiratoire Syncytial (VRS) est d'un vecteur, dans lequel le vecteur est une molécule qui est hétérologue à la petite protéine hydrophobe du Virus Respiratoire Syncytial (VRS) et dans lequel, en outre, ledit ectodomaine est chimiquement ou génétiquement lié au vecteur.

2. Vaccin destiné à être utilisé selon la revendication 1, dans lequel ledit ectodomaine se présente sous forme d'oligomère.

3. Vaccin destiné à être utilisé selon la revendication 1 ou 2, dans lequel ledit ectodomaine est constitué de SEQ ID N° 1 ou de SEQ ID N° 2.

4. Vaccin destiné à être utilisé selon la revendication 2, dans lequel ledit oligomère est un pentamère.

5. Vaccin destiné à être utilisé selon la revendication 1, dans lequel ledit vecteur est choisi dans le groupe constitué par la protéine matricielle oligomérique du cartilage (COMP), Lpp-56 et une pseudo-particule virale.

6. Vaccin destiné à être utilisé selon la revendication 1, dans lequel ledit vecteur est un vecteur non protéique.

7. Vaccin destiné à être utilisé selon la revendication 6, dans lequel ledit vecteur non protéique est un liposome ou un triméthyl-chitosane.

8. Anticorps spécifique de l'ectodomaine de la petite protéine hydrophobe du VRS, destiné à être utilisé dans un procédé de prévention ou de traitement d'une infection par le VRS.

9. Composition pharmaceutique, comprenant un anticorps monoclonal selon la revendication 8, destinée à être utilisée dans un procédé de prévention ou de traitement d'une infection par le VRS.


### SEQUENCE LISTING

<110> VIB VZW
   UNIVERSITEIT GENT
<120> RESPIRATORY SYNCYTIAL VIRUS VACCINE
<130> XaSa/RSV/359
<150> GB 1019240.9
   <151> 2010-11-15
<150> US 61/458,012
   <151> 2010-11-15
<160> 54
<170> PatentIn version 3.5
<210> 1
   <211> 23
   <212> PRT
   <213> respiratory syncytial virus
<400> 1
   Asn Lys Leu Cys Glu Tyr Asn Val Phe His Asn Lys Thr Phe Glu Leu
   1               5                   10                  15
   Pro Arg Ala Arg Val Asn Thr
               20
<210> 2
   <211> 24
   <212> PRT
   <213> respiratory syncytial virus
<400> 2
   Asn Lys Leu Ser Glu His Lys Thr Phe Cys Asn Asn Thr Leu Glu Leu
   1               5                   10                  15
   Gly Gln Met His Gln Ile Asn Thr
               20
<210> 3
   <211> 9
   <212> PRT
   <213> Artificial Sequence
<220>
   <223> Variant of ectodomain
<220>
   <221> MISC_FEATURE
   <222> (4)..(4)
   <223> Xaa can be Cys or Ser
<220>
   <221> MISC_FEATURE
   <222> (6)..(6)
   <223> Xaa can be Tyr or His
<220>
   <221> MISC_FEATURE
   <222> (7)..(7)
   <223> Xaa can be Asn or Lys
<220>
   <221> misc_feature
   <222> (8)..(8)
   <223> Xaa can be any naturally occurring amino acid
<400> 3
   Asn Lys Leu Xaa Glu Xaa Xaa Xaa Phe
   1               5
<210> 4
   <211> 23
   <212> PRT
   <213> respiratory syncytial virus
<400> 4
   Asn Lys Leu Cys Glu Tyr Asn Ile Phe His Asn Lys Thr Phe Glu Leu
   1               5                   10                  15
   Pro Arg Ala Arg Val Asn Thr
               20
<210> 5
   <211> 23
   <212> PRT
   <213> respiratory syncytial virus
<400> 5
   Asn Lys Leu Cys Glu Tyr Asn Val Phe His Asn Lys Thr Phe Glu Leu
   1               5                   10                  15
   Pro Lys Ala Arg Val Asn Thr
               20
<210> 6
   <211> 23
   <212> PRT
   <213> respiratory syncytial virus
<400> 6
   Asn Lys Leu Cys Glu Tyr Asn Val Phe Tyr Asn Lys Thr Phe Glu Leu
   1               5                   10                  15
   Pro Arg Ala Arg Val Asn Thr
                                 20
<210> 7
   <211> 23
   <212> PRT
   <213> respiratory syncytial virus
<400> 7
   Asn Lys Leu Cys Glu Tyr Asn Ala Phe His Asn Lys Thr Phe Glu Leu
   1               5                   10                  15
   Pro Arg Ala Arg Ile Asn Thr
               20
<210> 8
   <211> 24
   <212> PRT
   <213> respiratory syncytial virus
<400> 8
   Asn Lys Leu Ser Glu His Lys Thr Phe Cys Asn Lys Thr Leu Glu Leu
   1               5                   10                  15
   Gly Gln Met Tyr Gln Ile Asn Thr
               20
<210> 9
   <211> 24
   <212> PRT
   <213> respiratory syncytial virus
<400> 9
   Asn Lys Leu Ser Glu His Lys Thr Phe Cys Asn Lys Thr Leu Glu Gln
   1               5                   10                  15
   Gly Gln Met Tyr Gln Val Asn Thr
               20
<210> 10
   <211> 24
   <212> PRT
   <213> respiratory syncytial virus
<400> 10
   Asn Lys Leu Ser Glu His Lys Ala Phe Cys Asn Lys Thr Leu Glu Gln
   1               5                   10                  15
   Gly Gln Met Tyr Gln Ile Asn Thr
               20
<210> 11
   <211> 24
   <212> PRT
   <213> respiratory syncytial virus
<400> 11
   Asn Lys Leu Ser Glu His Lys Ile Phe Cys Asn Lys Thr Leu Glu Gln
   1               5                   10                  15
   Gly Gln Met Tyr Gln Ile Asn Thr
               20
<210> 12
   <211> 24
   <212> PRT
   <213> respiratory syncytial virus
<400> 12
   Asn Lys Leu Ser Glu His Lys Pro Phe Cys Asn Lys Thr Leu Glu Gln
   1               5                   10                  15
   Gly Gln Met Tyr Gln Ile Asn Thr
               20
<210> 13
   <211> 24
   <212> PRT
   <213> respiratory syncytial virus
<400> 13
   Asn Lys Leu Ser Glu His Lys Thr Phe Cys Asn Lys Thr Leu Glu Gln
   1               5                   10                  15
   Gly Gln Val Tyr Gln Ile Asn Thr
               20
<210> 14
   <211> 24
   <212> PRT
   <213> respiratory syncytial virus
<400> 14
   Asn Lys Leu Ser Glu His Lys Thr Phe Tyr Asn Lys Thr Leu Glu Gln
   1               5                   10                  15
   Gly Gln Met Tyr Gln Ile Asn Thr
               20
<210> 15
   <211> 24
   <212> PRT
   <213> respiratory syncytial virus
<400> 15
   Asn Lys Leu Ser Glu His Lys Ile Phe Cys Asn Lys Thr Leu Glu Gln
   1               5                   10                  15
   Gly Gln Met Tyr Gln Ile His Thr
               20
<210> 16
   <211> 24
   <212> PRT
   <213> respiratory syncytial virus
<400> 16
   Asn Lys Leu Ser Glu His Lys Thr Phe Phe Asn Lys Thr Leu Glu Gln
   1               5                   10                  15
   Gly Gln Met Tyr Gln Ile Asn Thr
               20
<210> 17
   <211> 40
   <212> PRT
   <213> respiratory syncytial virus
<400> 17
   Asn Lys Leu Cys Asp Phe Asn Asp His His Thr Asn Ser Leu Asp Ile
   1               5                   10                  15
   Arg Thr Arg Leu Arg Asn Asp Thr Gln Leu Ile Thr Arg Ala His Glu
               20                  25                  30
   Gly Ser Ile Asn Gln Ser Ser Asn
           35                  40
<210> 18
   <211> 14
   <212> PRT
   <213> Artificial Sequence
<220>
   <223> Variant of ectodomain
<220>
   <221> misc_feature
   <222> (5)..(9)
   <223> Xaa can be any naturally occurring amino acid
<400> 18
   Asn Lys Leu Cys Xaa Xaa Xaa Xaa Xaa His Thr Asn Ser Leu
   1 5 10
<210> 19
   <211> 40
   <212> PRT
   <213> respiratory syncytial virus
<400> 19
   Asn Lys Leu Cys Asp Phe Asn Asp Arg His Thr Asn Ser Leu Asp Ile
   1               5                   10                  15
   Arg Thr Arg Leu Arg Asp Asp Thr Gln Leu Ile Thr Arg Ala His Glu
               20                  25                  30
   Gly Ser Ile Asn Gln Ser Ser Asn
           35                  40
<210> 20
   <211> 40
   <212> PRT
   <213> respiratory syncytial virus
<400> 20
   Asn Lys Leu Cys Asp Leu Asn Asp His His Thr Asn Ser Leu Asp Ile
   1               5                   10                  15
   Arg Thr Arg Leu Arg Asn Asp Thr Gln Leu Thr Thr Arg Ala His Glu
               20                  25                  30
   Gly Pro Ile Asn Gln Ser Ser Asn
           35                  40
<210> 21
   <211> 40
   <212> PRT
   <213> respiratory syncytial virus
<400> 21
   Asn Lys Leu Cys Asp Leu Asn Asn His His Thr Asn Ser Leu Asp Ile
   1               5                   10                  15
   Arg Thr Arg Leu Arg Asn Asp Thr Gln Ser Ile Thr Arg Ala His Glu
               20                  25                  30
   Gly Ser Ile Asn Gln Ser Ser Asn
           35                  40
<210> 22
   <211> 40
   <212> PRT
   <213> respiratory syncytial virus
<400> 22
   Asn Lys Leu Cys Asp Leu Asn Asp His His Thr Asn Ser Leu Asp Ile
   1               5                   10                  15
   Arg Thr Lys Leu Arg Ser Asp Thr Gln Leu Ile Thr Arg Ala His Glu
               20                  25                  30
   Glu Ser Ile Asn Gln Ser Ser Asn
           35                  40
<210> 23
   <211> 40
   <212> PRT
   <213> respiratory syncytial virus
<400> 23
   Asn Lys Leu Cys Asp Leu Asn Asn His His Thr Asn Ser Leu Asp Ile
   1               5                   10                  15
   Lys Thr Arg Leu Arg Asn Asp Thr Gln Ser Ile Thr Arg Ala His Glu
               20                  25                  30
   Gly Ser Ile Asn Gln Ser Ser Asn
           35                  40
<210> 24
   <211> 40
   <212> PRT
   <213> respiratory syncytial virus
<400> 24
   Asn Lys Leu Cys Asp Leu Asn Asn His His Thr Asn Ser Leu Asp Ile
   1               5                   10                  15
   Lys Thr Arg Leu Lys Asn Asp Thr Gln Ser Ile Thr Arg Ala His Glu
               20                  25                  30
   Gly Ser Ile Asn Gln Ser Ser Asn
           35                  40
<210> 25
   <211> 40
   <212> PRT
   <213> respiratory syncytial virus
<400> 25
   Asn Lys Leu Cys Val Leu Asn Asn His His Thr Asn Ser Leu Asp Ile
   1               5                   10                  15
   Arg Thr Arg Leu Arg Asn Asp Thr Gln Ser Ile Thr Arg Ala His Glu
               20                  25                  30
   Gly Ser Ile Asn Gln Ser Ser Asn
           35                  40
<210> 26
   <211> 40
   <212> PRT
   <213> respiratory syncytial virus
<400> 26
   Asn Lys Leu Cys Asp Leu Asn Asn His His Thr Asn Ser Leu Glu Ile
   1               5                   10                  15
   Lys Thr Arg Leu Arg Asn Asp Thr Gln Ser Ile Thr Lys Ala His Glu
               20                  25                  30
   Gly Ser Ile Asn Gln Ser Ser Asn
           35                  40
<210> 27
   <211> 40
   <212> PRT
   <213> respiratory syncytial virus
<400> 27
   Asn Lys Leu Cys Asp Leu Asn Asn His His Thr Asn Ser Leu Asp Ile
   1               5                   10                  15
   Lys Thr Arg Leu Arg Asn Asp Thr Gln Ser Thr Thr Arg Ala His Glu
               20                  25                  30
   Gly Ser Ile Asn Gln Ser Ser Asn
           35                  40
<210> 28
   <211> 40
   <212> PRT
   <213> respiratory syncytial virus
<400> 28
   Asn Lys Leu Cys Val Leu Ser Asn His His Thr Asn Ser Leu Asp Ile
   1               5                   10                  15
   Arg Thr Arg Leu Arg Asn Asp Thr Gln Ser Ile Thr Arg Ala His Glu
               20                  25                  30
   Gly Ser Ile Asn Gln Ser Ser Asn
           35                  40
<210> 29
   <211> 36
   <212> PRT
   <213> respiratory syncytial virus
<400> 29
   Asn Lys Leu Cys Val Leu Asn Asn His His Thr Asn Ser Leu Asp Ile
   1               5                   10                  15
   Arg Thr Arg Leu Arg Asn Asp Thr Gln Ser Ile Thr Arg Ala His Glu
               20                  25                  30
   Gly Ser Ile Asn
           35
<210> 30
   <211> 31
   <212> PRT
   <213> respiratory syncytial virus
<400> 30
   Asn Lys Leu Cys Asp Leu Asn Asp His His Thr Asn Ser Leu Asp Ile
   1               5                   10                  15
   Arg Thr Gly Leu Arg Asn Asp Thr Gln Ser Ile Thr Arg Ala His
               20                  25                  30
<210> 31
   <211> 2973
   <212> DNA
   <213> Artificial Sequence
<220>
   <223> Plasmid puc57-comp5SHe
<220>
   <221> misc_feature
   <222> (440)..(442)
   <223> Startcodon reading frame Flag-COMPcc-SHe
<220>
   <221> misc_feature
   <222> (671)..(673)
   <223> Stopcodon reading frame Flag-COMPcc-SHe
<400> 31
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt acctcgcgaa 420
   tgcatctaga tattggcata tggattacaa agatgatgat gataaagatc tggccccaca 480
   gatgctgcgt gaactgcagg aaaccaatgc agccctgcag gatgttcgtg aactgctgcg 540
   tcaccgtgtg aaagaaatta ccttcctgaa aaatacggtc atggaatgtg acgcttgcgg 600
   caacaaactg tgcgaatata atgtttttca taataaaacc tttgaactgc ctcgtgcacg 660
   tgtgaacacc taaaagctta tgcatgcggc cgcattggga tcccgggccc gtcgactgca 720
   gaggcctgca tgcaagcttg gcgtaatcat ggtcatagct gtttcctgtg tgaaattgtt 780
   atccgctcac aattccacac aacatacgag ccggaagcat aaagtgtaaa gcctggggtg 840
   cctaatgagt gagctaactc acattaattg cgttgcgctc actgcccgct ttccagtcgg 900
   gaaacctgtc gtgccagctg cattaatgaa tcggccaacg cgcggggaga ggcggtttgc 960
   gtattgggcg ctcttccgct tcctcgctca ctgactcgct gcgctcggtc gttcggctgc 1020
   ggcgagcggt atcagctcac tcaaaggcgg taatacggtt atccacagaa tcaggggata 1080
   acgcaggaaa gaacatgtga gcaaaaggcc agcaaaaggc caggaaccgt aaaaaggccg 1140
   cgttgctggc gtttttccat aggctccgcc cccctgacga gcatcacaaa aatcgacgct 1200
   caagtcagag gtggcgaaac ccgacaggac tataaagata ccaggcgttt ccccctggaa 1260
   gctccctcgt gcgctctcct gttccgaccc tgccgcttac cggatacctg tccgcctttc 1320
   tcccttcggg aagcgtggcg ctttctcata gctcacgctg taggtatctc agttcggtgt 1380
   aggtcgttcg ctccaagctg ggctgtgtgc acgaaccccc cgttcagccc gaccgctgcg 1440
   ccttatccgg taactatcgt cttgagtcca acccggtaag acacgactta tcgccactgg 1500
   cagcagccac tggtaacagg attagcagag cgaggtatgt aggcggtgct acagagttct 1560
   tgaagtggtg gcctaactac ggctacacta gaagaacagt atttggtatc tgcgctctgc 1620
   tgaagccagt taccttcgga aaaagagttg gtagctcttg atccggcaaa caaaccaccg 1680
   ctggtagcgg tggttttttt gtttgcaagc agcagattac gcgcagaaaa aaaggatctc 1740
   aagaagatcc tttgatcttt tctacggggt ctgacgctca gtggaacgaa aactcacgtt 1800
   aagggatttt ggtcatgaga ttatcaaaaa ggatcttcac ctagatcctt ttaaattaaa 1860
   aatgaagttt taaatcaatc taaagtatat atgagtaaac ttggtctgac agttaccaat 1920
   gcttaatcag tgaggcacct atctcagcga tctgtctatt tcgttcatcc atagttgcct 1980
   gactccccgt cgtgtagata actacgatac gggagggctt accatctggc cccagtgctg 2040
   caatgatacc gcgagaccca cgctcaccgg ctccagattt atcagcaata aaccagccag 2100
   ccggaagggc cgagcgcaga agtggtcctg caactttatc cgcctccatc cagtctatta 2160
   attgttgccg ggaagctaga gtaagtagtt cgccagttaa tagtttgcgc aacgttgttg 2220
   ccattgctac aggcatcgtg gtgtcacgct cgtcgtttgg tatggcttca ttcagctccg 2280
   gttcccaacg atcaaggcga gttacatgat cccccatgtt gtgcaaaaaa gcggttagct 2340
   ccttcggtcc tccgatcgtt gtcagaagta agttggccgc agtgttatca ctcatggtta 2400
   tggcagcact gcataattct cttactgtca tgccatccgt aagatgcttt tctgtgactg 2460
   gtgagtactc aaccaagtca ttctgagaat agtgtatgcg gcgaccgagt tgctcttgcc 2520
   cggcgtcaat acgggataat accgcgccac atagcagaac tttaaaagtg ctcatcattg 2580
   gaaaacgttc ttcggggcga aaactctcaa ggatcttacc gctgttgaga tccagttcga 2640
   tgtaacccac tcgtgcaccc aactgatctt cagcatcttt tactttcacc agcgtttctg 2700
   ggtgagcaaa aacaggaagg caaaatgccg caaaaaaggg aataagggcg acacggaaat 2760
   gttgaatact catactcttc ctttttcaat attattgaag catttatcag ggttattgtc 2820
   tcatgagcgg atacatattt gaatgtattt agaaaaataa acaaataggg gttccgcgca 2880
   catttccccg aaaagtgcca cctgacgtct aagaaaccat tattatcatg acattaacct 2940
   ataaaaatag gcgtatcacg aggccctttc gtc 2973
<210> 32
   <211> 525
   <212> DNA
   <213> Artificial Sequence
<220>
   <223> mHBc expression vector
<400> 32
   gggcgaattg ggtaccggaa ttccatatgg atatcgatcc gtacaaagaa tttggcgcga 60
   ccgtggaact gctgtctttt ctgccgagcg atttttttcc gagcgtgcgt gatctgctgg 120
   ataccgcgag cgcgctgtat cgtgaagcgc tggaaagccc ggaacatagc agcccgcatc 180
   ataccgcgct gcgtcaggcg attctgtgct ggggcgaact gatgaccctg gccacctggg 240
   tgggcgtgaa cctggaagat ggcggcaaag gcggcagccg tgatctggtg gtgagctatg 300
   tgaacaccaa catgggcctg aaatttcgtc agctgctgtg gtttcatatc agcagcctga 360
   cctttggccg tgaaaccgtg ctggaatatc tggtgagctt tggcgtgtgg attcgtactc 420
   cgccggcata tcgtccgccg aacgcgccga ttctgagcac cctgccggaa accaccgtgg 480
   tgtgctagcg gccgcaaaag gaaaagagct ccagcttttg ttccc 525
<210> 33
   <211> 358
   <212> DNA
   <213> Artificial Sequence
<220>
   <223> SHe expression vector
<400> 33
   gggcgaattg ggtaccctgc agaaccacgt gggtgggtgt taacttggaa gatggcggca 60
   gcaacaaact gtgcgaatat aacgtgttcc acaataaaac ctttgaactg ccgcgtgcgc 120
   gtgtgaatac cagcggcggc agcggtggtt cgaataaact gtgtgaatac aatgtctttc 180
   ataacaagac gttcgaactg ccacgtgccc gcgtcaacac ctctggtggt agcggcggct 240
   ctaacaagtt atgtgagtac aacgtattcc acaacaagac atttgagttg cctcgggcac 300
   gagtaaatac atctggtggt gctagcaggg acctggtaga gctccagctt ttgttccc 358
<210> 34
   <211> 3250
   <212> DNA
   <213> Artificial Sequence
<220>
   <223> Template plasmid
<220>
   <221> misc_feature
   <222> (3150)..(3150)
   <223> n is a, c, g, or t
<400> 34
   atgaacaatg ctaccttcaa ctatacaaac gttaacccta tttctcacat caggggaggt     60
   ttaaaacaaa tcgaagacaa gctggaagaa atcctttcga aactgtacca catcgaaaac    120
   gagctggcca ggatcaagaa actgctgggc gaagaattcg aaggaatgga ctacaaggat    180
   catgatggtg attataaaga ccacgacatt gactataagg atgatgatga caaataggaa    240
   gcttatgcat gcggccgcat ctagagggcc cggatccctc gaggtcgacg aattcgagct    300
   cggccgactt ggccttccct ttagtgaggg ttaataaact tggtgagcaa taactagcat    360
   aaccccttgg ggcctctaaa cgggtcttga ggggtttttt gctgaaagga ggaactatat    420
   gcgctcatac gatatgaacg ttgagactgc cgctgagtta tcagtgagca ataactagca    480
   taaccccttg gggcctctaa acgggtcttg aggggttttt tgctgaaagg aggaactata    540
   tccggccgga tagcttatcg ctagaggtcg aaattcacct cgaaagcaag ctgataaacc    600
   gatacaatta aaggctcctt ttggagcctt tttttttgga gattttcaac gtgaaaaaat    660
   tattattcgc aattccaagc taattcacct cgaaagcaag ctgataaacc gatacaatta    720
   aaggctcctt ttggagcctt tttttttgga gattttcaac gtgaaaaaat tattattcgc    780
   aattccaagc tctgcctcgc gcgtttcggt gatgacggtg aaaacctctg acacatgcag    840
   ctccctaggc aattgcatgt gagcaaaagg ccagcaaaag gccaggaacc gtaaaaaggc 900
   cgcgttgctg gcgtttttcc ataggctccg cccccctgac gagcatcaca aaaatcgacg 960
   ctcaagtcag aggtggcgaa acccgacagg actataaaga taccaggcgt ttccccctgg 1020
   aagctccctc gtgcgctctc ctgttccgac cctgccgctt accggatacc tgtccgcctt 1080
   tctcccttcg ggaagcgtgg cgctttctca tagctcacgc tgtaggtatc tcagttcggt 1140
   gtaggtcgtt cgctccaagc tgggctgtgt gcacgaaccc cccgttcagc ccgaccgctg 1200
   cgccttatcc ggtaactatc gtcttgagtc caacccggta agacacgact tatcgccact 1260
   ggcagcagcc actggtaaca ggattagcag agcgaggtat gtaggcggtg ctacagagtt 1320
   cttgaagtgg tggcctaact acggctacac tagaaggaca gtatttggta tctgcgctct 1380
   gctgaagcca gttaccttcg gaaaaagagt tggtagctct tgatccggca aacaaaccac 1440
   cgctggtagc ggtggttttt ttgtttgcaa gcagcagatt acgcgcagaa aaaaaggatc 1500
   tcaagaagat cctttgatct tttctacggg gtctgacgct cagtggaacg aaaactcacg 1560
   ttaagggatt ttggtcatga gattatcaaa aaggatcttc acctagatcc ttttaaatta 1620
   aaaatgaagt tttaaatcaa tctaaagtat atatgagtaa acttggtctg acagttacca 1680
   atgcttaatc agtgaggcac ctatctcagc gatctgtcta tttcgttcat ccatagttgc 1740
   ctgactcccc gtcgtgtaga taactacgat acgggagggc ttaccatctg gccccagtgc 1800
   tgcaatgata ccgcgagacc cacgctcacc ggctccagat ttatcagcaa taaaccagcc 1860
   agccggaagg gccgagcgca gaagtggtcc tgcaacttta tccgcctcca tccagtctat 1920
   taattgttgc cgggaagcta gagtaagtag ttcgccagtt aatagtttgc gcaacgttgt 1980
   tgccattgct gcaggcatcg tggtgtcacg ctcgtcgttt ggtatggctt cattcagctc 2040
   cggttcccaa cgatcaaggc gagttacatg atcccccatg ttgtgcaaaa aagcggttag 2100
   ctccttcggt cctccgatcg ttgtcagaag taagttggcc gcagtgttat cactcatggt 2160
   tatggcagca ctgcataatt ctcttactgt catgccatcc gtaagatgct tttctgtgac 2220
   tggtgagtac tcaaccaagt cattctgaga atagtgtatg cggcgaccga gttgctcttg 2280
   cccggcgtca acacgggata ataccgcgcc acatagcaga actttaaaag tgctcatcat 2340
   tggaaaacgt tcttcggggc gaaaactctc aaggatctta ccgctgttga gatccagttc 2400
   gatgtaaccc actcgtgcac ccaactgatc ttcagcatct tttactttca ccagcgtttc 2460
   tgggtgagca aaaacaggaa ggcaaaatgc cgcaaaaaag ggaataaggg cgacacggaa 2520
   atgttgaata ctcatactct tcctttttca atattatgta agcagacagt tttattgttc 2580
   atgatgatat atttttatct tgtgcaatgt aacatcagag attttgagac acaacgtggc 2640
   tttgttgaat aaatcgaact tttgctgagt tgaaggatca gatcacgcat cttcccgaca 2700
   acgcagaccg ttccgtggca aagcaaaagt tcaaaatcac caactggtcc acctacaaca 2760
   aagctctcat caaccgtggc tccctcactt tctggctgga tgatggggcg attcaggcct 2820
   ggtatgagtc agcaacacct tcttcacgag gcagacctca gcgccggtga tgccggccac 2880
   gatgcgtccg gcgtagagga tctctcacct accaaacaat gcccccctgc aaaaaataaa 2940
   ttcatataaa aaacatacag ataaccatct gcggtgataa attatctctg gcggtgttga 3000
   cataaatacc actggcggtg atactgagca catcagcagg acgcactgac caccatgaag 3060
   gtgacgctct taaaattaag ccctgaagaa gggcagcatt caaagcagaa ggctttgggg 3120
   tgtgtgatac gaaacgaagc attggaattn cggatctcga tcccggaaat taatacgact 3180
   cactataggg agaccacaac ggtttccctc tagaaataat tttgtttaac tttaagaagg 3240
   agatatacat 3250
<210> 35
   <211> 77
   <212> PRT
   <213> Artificial Sequence
<220>
   <223> Vaccine Flag-COMPcc-SHe
<400> 35
   Met Asp Tyr Lys Asp Asp Asp Asp Lys Asp Leu Ala Pro Gln Met Leu
   1 5 10 15
   Arg Glu Leu Gln Glu Thr Asn Ala Ala Leu Gln Asp Val Arg Glu Leu
   20 25 30
   Leu Arg His Gln Val Lys Glu Ile Thr Phe Leu Lys Asn Thr Val Met
   35 40 45
   Glu Cys Asp Ala Cys Gly Asn Lys Leu Cys Glu Tyr Asn Val Phe His
   50 55 60
   Asn Lys Thr Phe Glu Leu Pro Arg Ala Arg Val Asn Thr
   65 70 75
<210> 36
   <211> 42
   <212> DNA
   <213> Artificial Sequence
<220>
   <223> Primer
<400> 36
   ataagaaagc ggccgctatg gaaaatacat ccataacaat ag 42
<210> 37
   <211> 41
   <212> DNA
   <213> Artificial Sequence
<220>
   <223> Primer
<400> 37
   gaagatctct atgtgttgac tcgagctctt ggtaactcaa a 41
<210> 38
   <211> 35
   <212> DNA
   <213> Artificial Sequence
<220>
   <223> Primer
<400> 38
   ggaattccat atgaacaagt tatgtgagta caacg 35
<210> 39
   <211> 42
   <212> DNA
   <213> Artificial Sequence
<220>
   <223> Primer
<400> 39
   gatttgtttt aaacctcctg tatttactcg tgcccgaggc aa 42
<210> 40
   <211> 23
   <212> PRT
   <213> respiratory syncytial virus
<400> 40
   Asn Lys Leu Cys Glu Tyr Asn Val Phe His Asn Lys Thr Phe Glu Leu
   1               5                   10                  15
   Pro Arg Ala Arg Val Asn Thr
               20
<210> 41
   <211> 36
   <212> DNA
   <213> Artificial Sequence
<220>
   <223> Primer
<400> 41
   cccaagcttc taacattgag attcccgaga ttgaga 36
<210> 42
   <211> 24
   <212> DNA
   <213> Artificial Sequence
<220>
   <223> Primer
<400> 42
   tattaaccct cactaaaggg aagg 24
<210> 43
   <211> 35
   <212> DNA
   <213> Artificial Sequence
<220>
   <223> Primer
<400> 43
   ggaattccat atgaacaagt tatgtgagta caacg 35
<210> 44
   <211> 24
   <212> DNA
   <213> Artificial Sequence
<220>
   <223> Primer
<400> 44
   tattaaccct cactaaaggg aagg 24
<210> 45
   <211> 20
   <212> DNA
   <213> Artificial Sequence
<220>
   <223> Primer
<400> 45
   tcacgaaggc tccacataca 20
<210> 46
   <211> 20
   <212> DNA
   <213> Artificial Sequence
<220>
   <223> Primer
<400> 46
   gcagggtcat cgtctttttc 20
<210> 47
   <211> 19
   <212> DNA
   <213> Artificial Sequence
<220>
   <223> Primer
<400> 47
   tgaagcaggc atctgaggg 19
<210> 48
   <211> 20
   <212> DNA
   <213> Artificial Sequence
<220>
   <223> Primer
<400> 48
   cgaaggtgga agagtgggag 20
<210> 49
   <211> 3252
   <212> DNA
   <213> Artificial Sequence
<220>
   <223> pLH36-HisDEVD-LPP₍₅₎-SHe plasmid
<400> 49
   gctgaaagga ggaactatat ccggccggat agcttatcgc tagaggtcga aattcacctc      60
   gaaagcaagc tgataaaccg atacaattaa aggctccttt tggagccttt ttttttggag      120
   attttcaacg tgaaaaaatt attattcgca attccaagct aattcacctc gaaagcaagc     180
   tgataaaccg atacaattaa aggctccttt tggagccttt ttttttggag attttcaacg      240
   tgaaaaaatt attattcgca attccaagct ctgcctcgcg cgtttcggtg atgacggtga     300
   aaacctctga cacatgcagc tccctaggca attgcatgtg agcaaaaggc cagcaaaagg     360
   ccaggaaccg taaaaaggcc gcgttgctgg cgtttttcca taggctccgc ccccctgacg     420
   agcatcacaa aaatcgacgc tcaagtcaga ggtggcgaaa cccgacagga ctataaagat     480
   accaggcgtt tccccctgga agctccctcg tgcgctctcc tgttccgacc ctgccgctta     540
   ccggatacct gtccgccttt ctcccttcgg gaagcgtggc gctttctcat agctcacgct     600
   gtaggtatct cagttcggtg taggtcgttc gctccaagct gggctgtgtg cacgaacccc     660
   ccgttcagcc cgaccgctgc gccttatccg gtaactatcg tcttgagtcc aacccggtaa     720
   gacacgactt atcgccactg gcagcagcca ctggtaacag gattagcaga gcgaggtatg     780
   taggcggtgc tacagagttc ttgaagtggt ggcctaacta cggctacact agaaggacag     840
   tatttggtat ctgcgctctg ctgaagccag ttaccttcgg aaaaagagtt ggtagctctt     900
   gatccggcaa acaaaccacc gctggtagcg gtggtttttt tgtttgcaag cagcagatta     960
   cgcgcagaaa aaaaggatct caagaagatc ctttgatctt ttctacgggg tctgacgctc    1020
   agtggaacga aaactcacgt taagggattt tggtcatgag attatcaaaa aggatcttca 1080
   cctagatcct tttaaattaa aaatgaagtt ttaaatcaat ctaaagtata tatgagtaaa 1140
   cttggtctga cagttaccaa tgcttaatca gtgaggcacc tatctcagcg atctgtctat 1200
   ttcgttcatc catagttgcc tgactccccg tcgtgtagat aactacgata cgggagggct 1260
   taccatctgg ccccagtgct gcaatgatac cgcgagaccc acgctcaccg gctccagatt 1320
   tatcagcaat aaaccagcca gccggaaggg ccgagcgcag aagtggtcct gcaactttat 1380
   ccgcctccat ccagtctatt aattgttgcc gggaagctag agtaagtagt tcgccagtta 1440
   atagtttgcg caacgttgtt gccattgctg caggcatcgt ggtgtcacgc tcgtcgtttg 1500
   gtatggcttc attcagctcc ggttcccaac gatcaaggcg agttacatga tcccccatgt 1560
   tgtgcaaaaa agcggttagc tccttcggtc ctccgatcgt tgtcagaagt aagttggccg 1620
   cagtgttatc actcatggtt atggcagcac tgcataattc tcttactgtc atgccatccg 1680
   taagatgctt ttctgtgact ggtgagtact caaccaagtc attctgagaa tagtgtatgc 1740
   ggcgaccgag ttgctcttgc ccggcgtcaa cacgggataa taccgcgcca catagcagaa 1800
   ctttaaaagt gctcatcatt ggaaaacgtt cttcggggcg aaaactctca aggatcttac 1860
   cgctgttgag atccagttcg atgtaaccca ctcgtgcacc caactgatct tcagcatctt 1920
   ttactttcac cagcgtttct gggtgagcaa aaacaggaag gcaaaatgcc gcaaaaaagg 1980
   gaataagggc gacacggaaa tgttgaatac tcatactctt cctttttcaa tattatgtaa 2040
   gcagacagtt ttattgttca tgatgatata tttttatctt gtgcaatgta acatcagaga 2100
   ttttgagaca caacgtggct ttgttgaata aatcgaactt ttgctgagtt gaaggatcag 2160
   atcacgcatc ttcccgacaa cgcagaccgt tccgtggcaa agcaaaagtt caaaatcacc 2220
   aactggtcca cctacaacaa agctctcatc aaccgtggct ccctcacttt ctggctggat 2280
   gatggggcga ttcaggcctg gtatgagtca gcaacacctt cttcacgagg cagacctcag 2340
   cgccggtgat gccggccacg atgcgtccgg cgtagaggat ctctcaccta ccaaacaatg 2400
   cccccctgca aaaaataaat tcatataaaa aacatacaga taaccatctg cggtgataaa 2460
   ttatctctgg cggtgttgac ataaatacca ctggcggtga tactgagcac atcagcagga 2520
   cgcactgacc accatgaagg tgacgctctt aaaattaagc cctgaagaag ggcagcattc 2580
   aaagcagaag gctttggggt gtgtgatacg aaacgaagca ttggaattcc ggatctcgat 2640
   cccggaaatt aatacgactc actataggga gaccacaacg gtttccctct agaaataatt 2700
   ttgtttaact ttaagaagga gatatacata tgcatcatca ccatcaccat cccggcggct 2760
   cggacgaagt ggatgcgaaa tgggatcagt ggagcagcga ttggcagacc tggaacgcga 2820
   aatgggatca gtggagcaac gattggaacg cgtggcgcag cgattggcag gcgtggaaag 2880
   atgattgggc gcgctggaac cagcgctggg ataactgggc gaccggcggc aacaaactgt 2940
   gcgaatataa cgtgtttcat aacaaaacct ttgaactgcc gcgcgcgcgc gtgaacacct 3000
   agggatccct cgaggtcgac gaattcgagc tcggccgact tggccttccc tttagtgagg 3060
   gttaataaac ttggtgagca ataactagca taaccccttg gggcctctaa acgggtcttg 3120
   aggggttttt tgctgaaagg aggaactata tgcgctcata cgatatgaac gttgagactg 3180
   ccgctgagtt atcagtgagc aataactagc ataacccctt ggggcctcta aacgggtctt 3240
   gaggggtttt tt 3252
<210> 50
   <211> 28
   <212> PRT
   <213> respiratory syncytial virus
<400> 50
   Cys Gly Gly Gly Ser Asn Lys Leu Ser Glu Tyr Asn Val Phe His Asn
   1 5 10 15
   Lys Thr Phe Glu Leu Pro Arg Ala Arg Val Asn Thr
   20 25
<210> 51
   <211> 29
   <212> PRT
   <213> respiratory syncytial virus
<400> 51
   Cys Gly Gly Gly Ser Asn Lys Leu Ser Glu His Lys Thr Phe Ser Asn
   1 5 10 15
   Lys Thr Leu Glu Gln Gly Gln Met Tyr Gln Ile Asn Thr
   20 25
<210> 52
   <211> 90
   <212> PRT
   <213> Artificial Sequence
<220>
   <223> chimeric LPP(5)-SHe protein
<400> 52
   Met His His His His His His Pro Gly Gly Ser Asp Glu Val Asp Ala
   1               5                   10                  15
   Lys Trp Asp Gln Trp Ser Ser Asp Trp Gln Thr Trp Asn Ala Lys Trp
               20                  25                  30
   Asp Gln Trp Ser Asn Asp Trp Asn Ala Trp Arg Ser Asp Trp Gln Ala
           35                  40                  45
   Trp Lys Asp Asp Trp Ala Arg Trp Asn Gln Arg Trp Asp Asn Trp Ala
       50                  55                  60
   Thr Gly Gly Asn Lys Leu Cys Glu Tyr Asn Val Phe His Asn Lys Thr
   65                  70                  75                  80
   Phe Glu Leu Pro Arg Ala Arg Val Asn Thr
                   85                  90
<210> 53
   <211> 24
   <212> DNA
   <213> Artificial Sequence
<220>
   <223> Primer
<400> 53
   gcgaaatggg atcagtggag cagc 24
<210> 54
   <211> 36
   <212> DNA
   <213> Artificial Sequence
<220>
   <223> Primer
<400> 54
   aatataggat ccctaggtcg cccagttatc ccagcg 36